# EUROPEAN PATENT SPECIFICATION

(11) **EP 1 547 545 B1**
(45) Date of publication and mention of the grant of the patent: **03.06.2009**
(21) Application number: 04030598.9
(22) Date of filing: 23.12.2004
(51) Int. Cl.: A61F 2/01, A61B 17/00, A61B 17/12

(54) **Embolus capturing device and implement for deployment of the same**
Embolusfangvorrichtung und Anordnung zum Anbringen desselben
Dispositif de capture d'embole et arrangement pour le déployer de ce dernier

(30) Priority: 26.12.2003 JP 2003434583
(43) Date of publication of application: 29.06.2005
(73) Proprietor: TERUMO KABUSHIKI KAISHA, Tokyo (JP)
(72) Inventor: Kanazawa, Susumu, Okayama-ken (JP); Sugiu, Kenji, Okayama-ken (JP); Numata, Miho, Okayama-shi Okayama-ken (JP); Murata, Yukuhiko, Tokyo (JP)
(74) Representative: Casalonga, Axel

(56) References cited:
- EP-A- 0 864 301
- WO-A-00/44308
- WO-A-01/72239
- WO-A-02/22048
- WO-A-03/077984
- DE-C1- 19 509 464

## Description

### BACKGROUND OF THE INVENTION

The present invention relates to an embolus member capturing device and an embolus member capturing device leaving implement for capturing, at a target occlusion site of a blood vessel, of an embolus member used in blood vessel embolization in the cases of abdominal bloodstream alteration, A-V shunt (A-P shunt), digestive tract bleeding, lung AVM, etc.

Conventionally, in hepatic artery injection therapy, for example, a carcinostatic is injected through an artery to a tumor site in order to cure primary hepatoma or metastatic hepatoma. In such a case, the above-mentioned blood vessel embolization is used. Specifically, the gastroduodenal artery, the right gastric artery, and/or the left gastric artery is occluded by an embolus metallic coil or the like, for preventing the carcinostatic from flowing into the gastroduodenal artery which is branched from the common hepatic artery. This is because if the carcinostatic flows into the gastroduodenal artery or the like, the carcinostatic would give side effects to the stomach and/or the duodenum, resulting in ulceration. Therefore, in some cases, a catheter is inserted into the gastroduodenal artery, and an embolus member is introduced through the lumen of the catheter and is left to indwell in a part of the gastroduodenal artery for generating an embolus there, thereby stopping the bloodstream. Examples of the embolus member include those of the liquid type and those of the particle type, in addition to the metallic coil type mentioned above. Examples of the metallic coil include those having the respective diameters of 0.25, 0.46, 0.53, 0.63 and 0.89mm (0.010, 0.018, 0.021, 0.025, and 0.035 inches), with a length of from 5 to 60 mm. When pushed out of the catheter, the metallic coil is left to indwell in the blood vessel in the state of being in a prememorized shape such as a spiral shape, a flower-like shape, a C-shape, and a straight shape. Further, there are metallic coils of the type of being fitted with a thread or threads for promoting thrombus formation. Such a metallic coil type embolus member is left to indwell in the blood vessel after pushed out of the catheter by use of a pusher.

In addition, devices have been provided in which a metallic coil is joined to the tip end of a guide wire form device so that the metallic coil can be separated electrically or mechanically (US Patent Nos. 5122136 and 5250071).

Besides, for capturing an embolus metallic coil in a blood vessel, a device disclosed in Japanese Patent Laid-open No. 2000-245739, for example, has been proposed. In this device, radially expandable spring legs are formed by bending one or a plurality of flexible wires such as stainless wires and are so connected as to be in an M shape through a proximal end connection portion. This device in a folded form is inserted into the lumen of the catheter from a proximal end portion of the catheter, is then moved to the distal end of the catheter, and the catheter is introduced to a target blood vessel. Thereafter, the device is pushed out into the blood vessel by a pushing means, whereon the once folded radially expandable spring legs are expanded, whereby the device (hence, the embolus metallic coil) is left to indwell in the blood vessel.

However, where only one embolus metallic coil is left to indwell in the blood vessel, it is often impossible to occlude the blood vessel. To be more specific, since the density of the coil at the planned occlusion portion is not high, the thrombus-forming effect and the bloodstream-interrupting effect are insufficient In such a case, it is necessary to add the metallic coils until the blood vessel is satisfactorily occluded. In addition, once the metallic coil is pushed out of the catheter, it is extremely difficult to pull the metallic coil backwards. Furthermore, the indwelling position of the embolus metallic coil is not stable. For example, when it is intended to guide the metallic coil through the catheter and to leave the metallic coil to indwell at a target position, the metallic coil may be unintentionally flowed downstream. As a result of these difficulties, it takes a long time to complete the intended occlusion of a blood vessel.

The devices of the electrically or mechanically separable type as disclosed in US Patent Nos. 5122136 and 5250071 also have the problem that once the metallic coil is separated from the guide wire form device, the position of the metallic coil cannot be changed. Besides, when the density of the metallic coil is not high enough to provide sufficient occlusion of a blood vessel, it is necessary to newly add the metallic coils.

The device as disclosed in Japanese Patent Laid-open No. 2000-245739 has a problem as to the stability of the indwelling position, like in the case of the metallic coil.

Reference can also be made to WO 03/077 984 directed to an implantable lumen occluding device causing rapid occlusion of a vessel lumen. A device having all the features of the preamble of claim 1 is known from WO 02/22048.

### SUMMARY OF THE INVENTION

The invention provides an embolus member capturing device leaving implement comprising a catheter having an expandable and foldable expansion body in the vicinity of a distal end portion, and an embolus member capturing device which is mounted to said catheter and is comprised of a tubular body having one end portion and the other end portion, wherein said expansion body comprises a proximal side expandable portion, which is cylindrical and is expandable to a first outside diameter, a distal side expandable portion expandable to a second outside diameter smaller than said first outside diameter, and an intermediate expandable portion expandable to a shape reduced in diameter toward the distal side between said proximal side expandable portion and said distal side expandable portion; and said embolus member capturing device is mounted to said expansion body in such a manner that said other end portion of said capturing device envelopes said proximal side expandable portion, said one end portion of said capturing device envelopes said distal side expandable portion, and the portion between said one end portion and said other end portion of said capturing device envelopes said intermediate expandable portion.

### BRIEF DESCRIPTION OF THE DRAWINGS

These and other objects of the invention will be seen by reference to the description, taken in connection with the accompanying drawing, in which:
Fig. 1 is a perspective view of one possible construction of an embolus member capturing device (embolus coil capturing device), which does not form a part of the present invention;
Fig. 2 is a perspective view showing the condition where the embolus member capturing device shown in Fig. 1 is expanded and an embolus member is thereby captured;
Fig. 3 is a front view of another possible construction of the embolus member capturing device which does not form a part of the present invention;
Fig. 4 is a development of the embolus member capturing device shown in Fig. 3;
Fig. 5 is a front view of a further possible construction of the embolus member capturing device which does not form a part of the present invention;
Fig. 6 is a development of the embolus member capturing device shown in Fig. 5;
Fig. 7 is a front view of yet another possible construction of the embolus member capturing device which does not form a part of the present invention;
Fig. 8 is a development of the embolus member capturing device shown in Fig. 7;
Fig. 9 is a front view of one possible construction of an embolus member capturing device leaving implement which does not form a part of the present invention;
Fig. 10 is a partly broken enlarged view of a distal end portion of the embolus member capturing device leaving implement shown in Fig. 9;
Fig. 11 is a view showing an expanded state of an expansion body in the embolus member capturing device leaving implement in the condition where an embolus member capturing device is not mounted;
Fig. 12 is a partly broken enlarged view of a proximal end portion of the embolus member capturing device leaving implement shown in Fig. 9;
Fig. 13 is a front view of another possible construction of the embolus member capturing device leaving implement which does not form a part of the present invention;
Fig. 14 is a view showing the condition where a sheath has been removed from the embolus member capturing device leaving implement shown in Fig. 13;
Fig. 15 is an enlarged sectional view of a central joint portion of the embolus member capturing device leaving implement shown in Fig. 13;
Fig. 16 is an end view along line A-A of Fig. 13;
Fig. 17 is an end view along line B-B of Fig. 13;
Fig. 18 is a sectional view along line C-C of Fig. 15;
Fig. 19 is a sectional view along line D-D of Fig. 15;
Fig. 20 is an enlarged sectional view of a proximal portion of the embolus member capturing device leaving implement shown in Fig. 13;
Fig. 21 is an enlarged sectional view of a proximal portion of a catheter used in the embolus member capturing device leaving implement shown in Figs. 13 and 14;
Fig. 22 is a front view of a further possible construction of the embolus member capturing device leaving implement according to the present invention;
Fig. 23 is a partly broken enlarged view of a distal end portion of the embolus member capturing device leaving implement shown in Fig. 22;
Fig. 24 is a view showing an expanded state of an expansion body of the embolus member capturing device leaving implement in the condition where an embolus member capturing device is not mounted;
Fig. 25 is a view showing the condition where the embolus member capturing device is expanded by the expansion body of the embolus member capturing device leaving implement;
Fig. 26 is a view showing an expanded state of the expansion body in the condition where an embolus member capturing device is not mounted, in an embolus member capturing device leaving implement according to an embodiment of the present invention;
Fig. 27 is a view showing the condition where the embolus member capturing device in the embolus member capturing device leaving implement according to the embodiment shown in Fig. 26 is expanded by the expansion body;
Fig. 28 is a front view of one example of the embolus member capturing device used in the embolus member capturing device leaving implement according to the present invention;
Fig. 29 is a front view of one example of the embolus member capturing device used in the embolus member capturing device leaving implement according to the present invention;
Fig. 30 is a front view of one example of the embolus member capturing device used in the embolus member capturing device leaving implement according to the present invention;
Fig. 31 is a front view of one example of the embolus member capturing device used in the embolus member capturing device leaving implement according to the present invention;
Fig. 32 is a perspective view of an possible construction of a blood vessel embolus member which does not form a part of the present invention;
Fig. 33 is a view showing the condition where the blood vessel embolus member is expanded by the expansion body of the embolus member capturing device;
Fig. 34 is a perspective view of a blood vessel embolus member according to another embodiment of the present invention;
Fig. 35 is an illustration of the condition where the blood vessel embolus member according to another embodiment of the present invention is mounted to an embolus member leaving implement;
Fig. 36 is an enlarged sectional view of a distal end portion of an embolus member leaving catheter used in Fig. 35; and
Fig. 37 is a perspective view of a further possible construction of a blood vessel embolus member, which does not form a part of the present invention;
Figs. 38 to 42 show a process for leaving the embolus member capturing device to indwell in the site of a blood vessel.

### DETAILED DESCRIPTION OF THE PREFERRED EMBODIMENTS

Now, an embolus member capturing device according to the present invention will be described using an embodiment shown in the drawings.

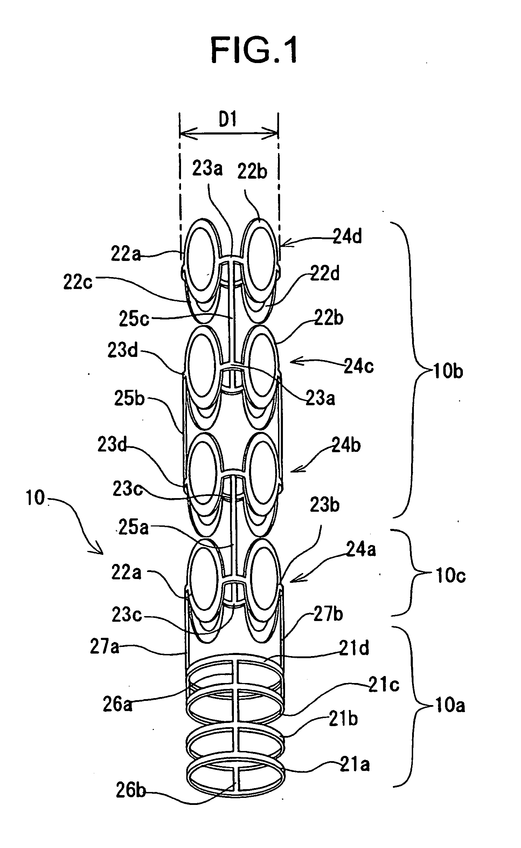

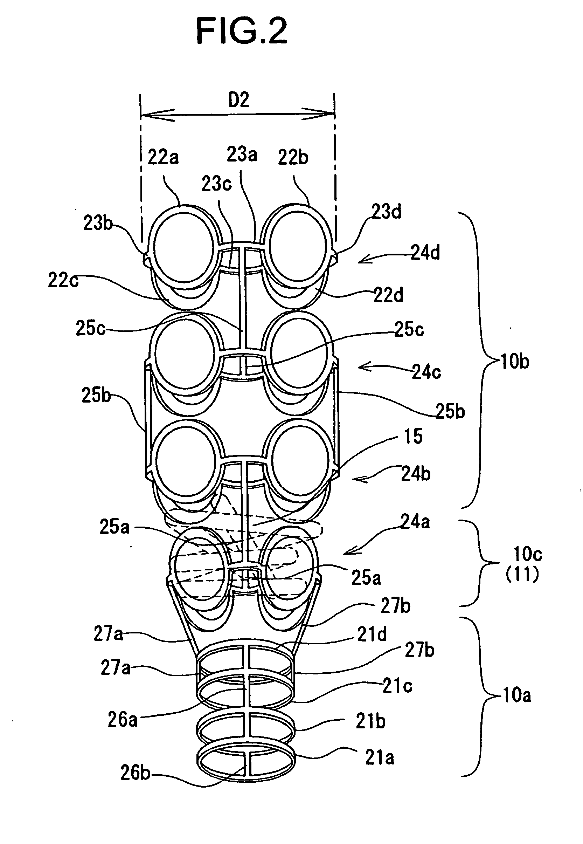

Fig. 1 is a perspective view of one possible construction of the embolus member capturing device (embolus coil capturing device), which does not form a part the present invention. Fig. 2 is a perspective view showing the condition where the embolus member capturing device shown in Fig. 1 is expanded and an embolus member is captured.

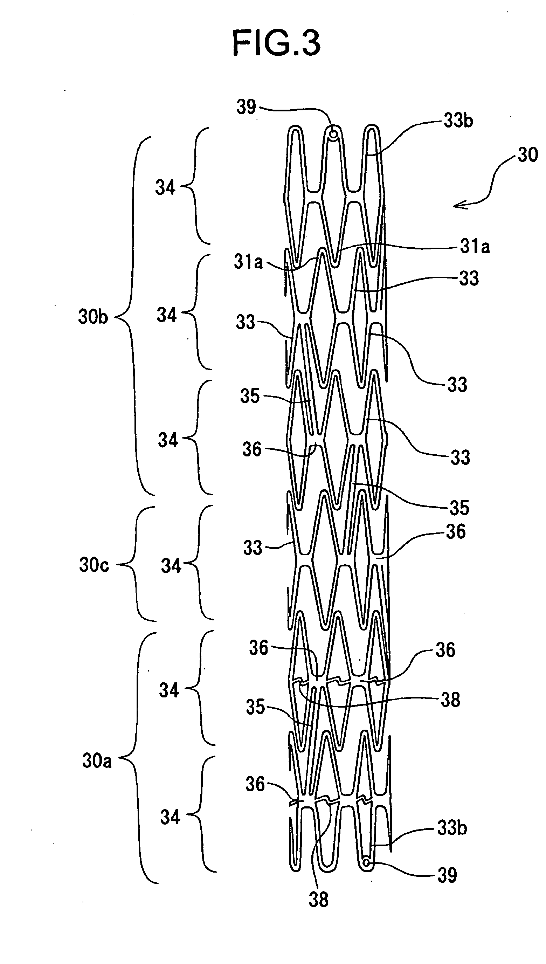

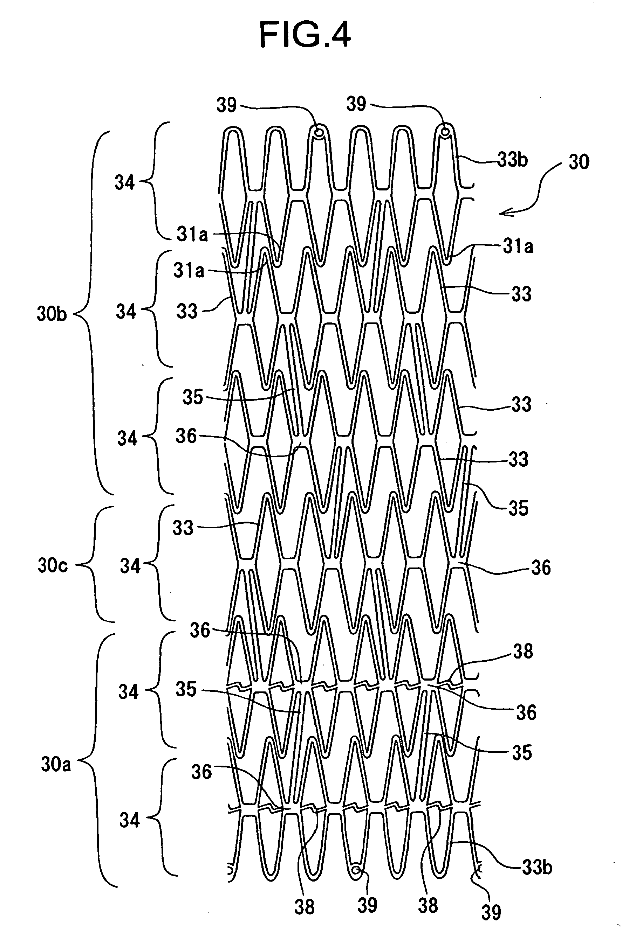

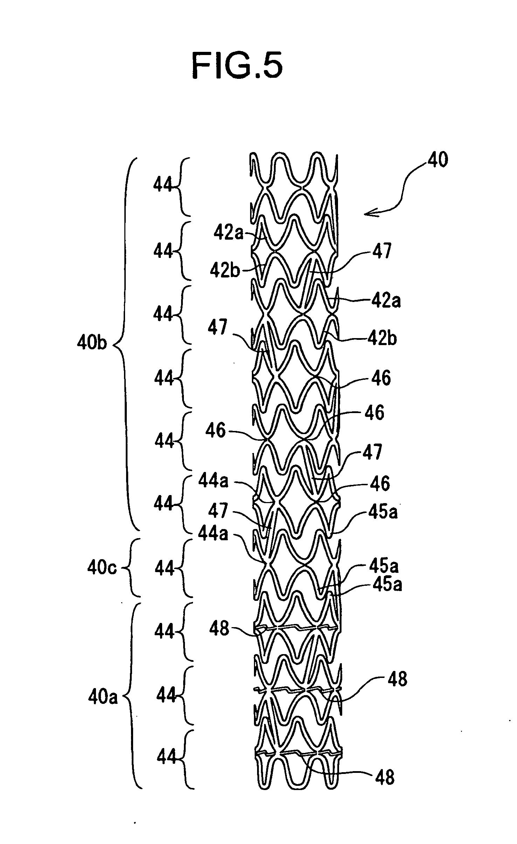

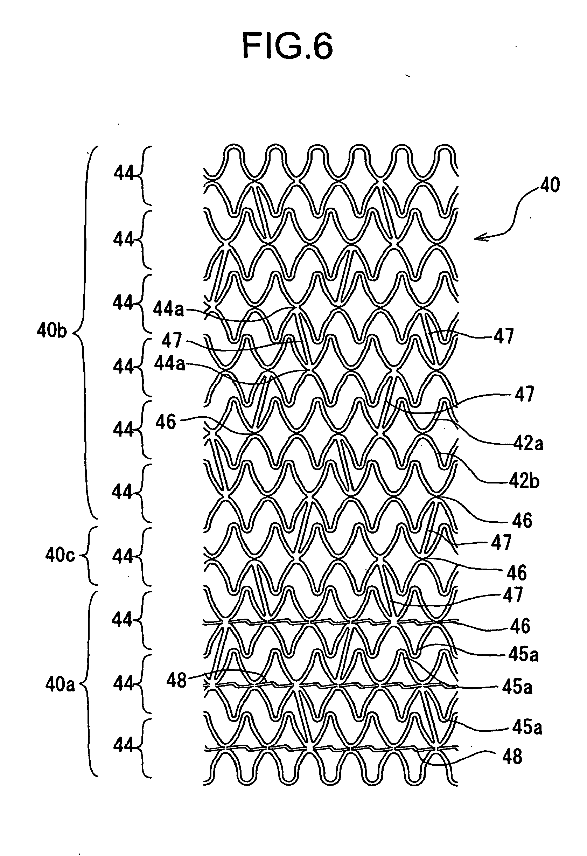

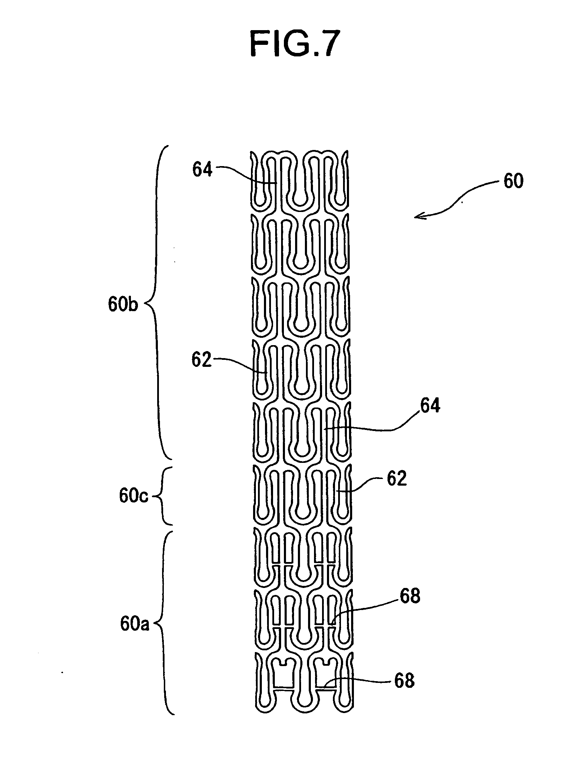

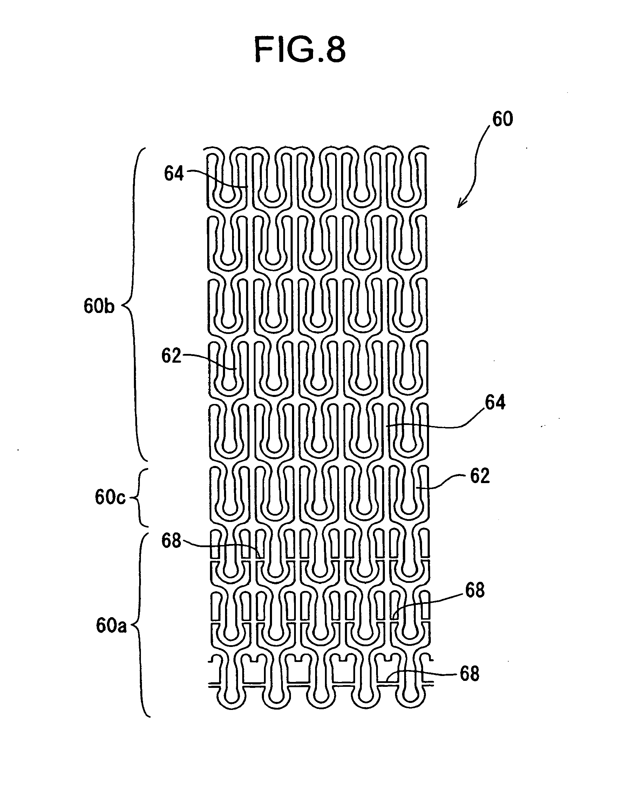

Fig. 3 is a front view of another possible construction of the embolus member capturing device. Fig. 4 is a development of the embolus member capturing device shown in Fig. 3. Fig. 5 is a front view of a further possible construction of the embolus member capturing device. Fig. 6 is a development of the embolus member capturing device shown in Fig. 5. Fig. 7 is a front view of yet another possible construction of the embolus member capturing device. Fig. 8 is a development of the embolus member capturing device shown in Fig. 7.

The embolus member capturing device 10, which does not form a part of the present invention is a tubular body having one end portion 10a and the other end portion 10b, and is expandable when a radially dilating force is exerted from the inside of the tubular body. The other end portion 10b of the embolus member capturing device 10 can make close contact with a blood vessel wall through expansion of the outside diameter thereof when a radially dilating force is exerted from the inside of the tubular body, whereas the one end portion 10a is slightly expanded from the outside diameter D1 before expansion thereof or substantially maintains the outside diameter D1 before expansion thereof when a radially dilating force is exerted from the inside of the tubular body, and either the one end portion 10a or an intermediate portion 10c between the one end portion 10a and the other end portion 10b forms an embolus member capturing portion 11.

In other words, the embolus member capturing device 10 is a tubular body having the one end portion 10a and the other end portion 10b and having a substantially constant first outside diameter D1, and is expandable when a radially dilating force is exerted from the inside of the tubular body, wherein the other end portion 10b of the embolus member capturing device 10 is expanded to a second outside diameter D2 larger than the first outside diameter D1 when a radially dilating force is exerted from the inside of the tubular body, whereas the one end portion 10a is slightly expanded from the first outside diameter D1 or substantially maintains the first outside diameter D1 when a radially dilating force is exerted from the inside of the tubular body, and either the one end portion 10a or the intermediate portion 10c between the one end portion 10a and the other end portion 10b forms the embolus member capturing portion 11.

Incidentally, the intermediate portion 10c preferably forms an embolus member capturing portion having an inside diameter varying from the one end side toward the other end portion upon expansion of an expansion body.

The embolus member capturing device 10 in this possible construction is formed to be a tubular body, and has the first diameter D1 suitable for insertion thereof into a blood vessel. The intermediate portion 10c and the other end portion 10b of the embolus member capturing device 10 are expandable (extensible) when a radially dilating force is exerted from the inside of the tubular body, i.e., when the expansion body is expanded. In the embolus member capturing device 10 according to this possible construction, as shown in Fig. 1, the intermediate portion 10c and the other end portion 10b are composed of annular units 24 (24a, 24b, 24c, 24d) in each of which elliptic or polygonal (in the figure, elliptic) component elements 22 (22a, 22b, 22c, 22d) elongate in the axial direction of the embolus member capturing device 10 and closed at central portions thereof are arranged on a circular circumference at substantially equal angular intervals in relation to the center axis of the embolus member capturing device 10, and adjacent portions (side portions) in the circumferential direction of the component elements are connected through connection portions 23 (23a, 23b, 23c, 23d); a plurality of the annular units 24a, 24b, 24c, 24d are arrayed in the axial direction of the embolus member capturing device 10. Further, it is preferable that the connection portion(s) 23 of the annular unit 24 is connected to the connection portion(s) 23 of the adjacent annular unit 24 through a link portion or link portions 25 (25a, 25b, 25c) with at least one location.

The one end portion 10a of the embolus member capturing device 10 is non-expandable (non-extensible) even when a radially dilating force is exerted from the inside of the tubular body, i.e., when the expansion body is expanded. Specifically, the one end portion 10a includes a plurality of annular bodies 21 (21a, 21b, 21c, 21d) substantially orthogonal to the center axis of the embolus member capturing device 10. The plurality of annular bodies 21a, 21b, 21c, 21d are arranged substantially in parallel to the axial direction of the embolus member capturing device 10. Further, the plurality of annular bodies 21a, 21b, 21c, 21d are linked by link portions 26 (26a, 26b). The link portions 26 may be provided in plurality as in this possible construction, or only one link portion may be provided. Incidentally, the link portions 26 are not limited to those for linking all the annular bodies rectilinearly as in this possible construction, and may be so provided as to link the adjacent annular bodies at different positions. The one end portion 10a is linked to the intermediate portion 10c by link portions 27 (27a, 27b). The link portions 27 may be provided in plurality as in this possible construction, or only one link portion may be provided. Incidentally, the one end portion 10a is not limited to the one configured as above-described, and may have any configuration inasmuch as it is non-expandable (non-extensible) when the expansion body is expanded; for example, the one end portion 10a is in the shape of a short pipe.

As shown in Fig. 2, the embolus member capturing device 10 is expanded by the expansion body; in this case, since the one end portion 10a is formed of the annular bodies and is therefore non-expandable, only the other end portion 10b and the intermediate portion 10c are expanded, whereon the other end portion 10b has the outside diameter D2. The outside diameter D2 is equal to or slightly greater than the inside diameter of the blood vessel in which the embolus member capturing device is to be left to indwell. Since the intermediate portion 10c is connected to the annular body 21d constituting the one end portion 10a through the link portions 27 (27a, 27b), the intermediate portion 10c cannot be sufficiently expanded and is expanded into a conical shape to thereby form an embolus member capturing portion. Incidentally, the embolus member may be, for example, an embolus coil; in Fig. 2, the condition where an embolus coil 15 is captured by an embolus member capturing portion formed of the intermediate portion 10b being radially enlarged is indicated by broken lines.

The embolus member capturing device may be in the form as shown in Figs. 3 and 4. Fig. 3 is a front view of another possible construction of the embolus member capturing device. Fig. 4 is a development of the embolus member capturing device shown in Fig. 3.

The embolus member capturing device 30 in this possible construction is formed to be a tubular body, has a diameter suitable for insertion thereof into a blood vessel, and is expandable when a radially dilating force is exerted from the inside of the tubular body. The embolus member capturing device 30 is composed of annular units 34 in each of which a plurality of polygonal filamentous bodies 33 each having a multiplicity of filamentous bent portions and an opening so as to be extended when a radially dilating force is exerted thereon are connected through a plurality of connection portions 36 so as to form an annular shape, wherein a plurality of the annular units 34 are arranged in the axial direction of the embolus member capturing device 30. In addition, the embolus member capturing device 30 includes link portions 35 which link the adjacent annular units 34 to each other at the connection portions and which are not in continuity in the axial direction of the embolus member capturing device. Further, the link portions 35 are provided between the adjacent annular units 34 in plurality and at opposite positions or at substantially equal angular intervals in relation to the center axis of the embolus member capturing device.

The embolus member capturing device is a so-called balloon expandable embolus member capturing device.

The polygonal filamentous bodies 33 located in one end portion 30a of the embolus member capturing device 30 are provided with extension restrictive portions 38 for restricting extension thereof when a radially dilating force is exerted thereon. Therefore, the one end portion 30a of the embolus member capturing device 30 is slightly enlarged in outside diameter or is substantially unchanged in outside diameter when a radially dilating force is exerted from the inside of the tubular body. On the other hand, the polygonal filamentous bodies 33 located in the other end portion 30b are extended when a radially dilating force is exerted thereon, resulting in that the outside diameter of the other end portion 30b is enlarged. The polygonal filamentous bodies 33 located in an intermediate portion 30c are not provided with any extension restrictive portion, so that they are extensible when a radially dilating force is exerted thereon; however, they are linked to the annular unit 34 constituting the one end portion 30a through the link portions 35, so that the expansion thereof is restricted, and an increase in outside diameter of the intermediate portion 30c is smaller than that of the other end portion 30b. As a result, upon expansion, the outside diameter of the intermediate portion 30c of the embolus member capturing device 30 lies between the outside diameter of the one end portion 30a and the outside diameter of the other end portion 30b. In the embolus member capturing device 30, either the intermediate portion 30c or the one end portion 30a forms an embolus member capturing portion, upon expansion.

Though the extension restrictive portions 38 may be in a rectilinear shape, but it is preferable for the extensive restrictive portions 38 to be curved or bent so as to allow some expansion of the one end portion 30a. The curved or bent shape is not limited to the zigzag shape as shown in Figs. 3 and 4; for example, a gradual arcuate shape or a wedge-like shape may be adopted.

In the embolus member capturing device according to this possible construction, the polygonal filamentous bodies 33 are in a collapsed shape elongate in the axial direction of the embolus member capturing device. Further, as in the embolus member capturing device according to this possible construction, each of the polygonal filamentous bodies 33 located at both ends of the embolus member capturing device is preferably so shaped that its portion located on the outside is semi-elliptic in shape. Furthermore, as in the embolus member capturing device in this possible construction, it is preferable that apex portions of the bent portions 31a of the polygonal filamentous bodies 33 in each annular unit 34 lie in the spaces formed between the bent portions 31a of the adjacent polygonal filamentous bodies 33 in the adjacent annular unit 34.

As shown in Fig. 4 which is a development of Fig. 3, the embolus member capturing device 30 is composed of the annular units 34 in each of which the polygonal filamentous bodies 33 elongate in the axial direction of the embolus member capturing device 30 and each having filamentous bent portions 31a and a central opening are arranged on the circular circumference at substantially equal angular intervals in relation to the center axis of the embolus member capturing device 30, and the adjacent portions (side portions) in the circumferential direction of the polygonal filamentous bodies 33 are connected to each other through the connection portions 36, wherein a plurality of the annular units 34 are arrayed in the axial direction of the embolus member capturing device 30. Further, the connection portions 36 in one annular unit 34 and the connection portion 36 in the adjacent annular unit 34 are linked through link portions 35 at least two or more locations. In other words, the embolus member capturing device 30 is a tubular body having a configuration in which a multiplicity of annular units 34 are linked to each other through the link portions 35.

In this possible construction, the annular unit 34 has six polygonal filamentous bodies arranged at substantially equal angular intervals. Each of the polygonal filamentous bodies 33 is formed in a rhombic shape elongate in the axial direction of the embolus member capturing device 30, and has a structure in which a central portion is opened in a rhombic shape corresponding to the shape of the polygonal filamentous body 33, and both end portions in the axial direction of the embolus member capturing device 30 are the filamentous bent portions 31a. Thus, each of the polygonal filamentous bodies 33 is so shaped as to form an individually independent closed system; in other words, each of the polygonal filamentous bodies 33 is a ring-shaped element which opens in a side surface of the embolus member capturing device 30. Such a shape of the polygonal filamentous bodies 33 promises a strong expansion holding force. In addition, each of the polygonal filamentous bodies 33 is curved in the circular circumferential direction so that the whole part thereof is substantially equally spaced from the center axis of the embolus member capturing device 30.

The center of a side portion, in the axial direction of the embolus member capturing device 30, of each polygonal filamentous body 33 and the center of a side portion, in the axial direction, of the adjacent polygonal filamentous body 33 are connected to each other through the short connection portion 36. That is to say, the connection portions 36 connect the polygonal filamentous bodies 33 in the circular circumferential direction. Since the connection portions 36 are substantially unchanged even when the embolus member capturing device 30 is expanded, the expanding force is liable to be exerted on the center of each polygonal filamentous body 33, so that each polygonal filamentous body 33 can be expanded (deformed) uniformly.

The number of the polygonal filamentous bodies 33 in each annular unit 34 is not limited to six, and is preferably in the range of four to eight Besides, the shape of the polygonal filamentous bodies 33 is preferably a polygonal shape having apexes which are opposite to each other in the axial direction of the embolus member capturing device 30, and may particularly be a rhombic shape, a hexagonal shape, an octagonal shape, or the like. Preferably, the shape is a rhombic shape, in view of the stability of deformation at the time of expansion of the embolus member capturing device 30.

The connection portions 36 in each annular unit 34 and the connection portions 36 in the adjacent annular unit 34 are linked through the link portions 35 which are formed to be comparatively long (longer than the connection portions 36) and parallel to the axial direction of the embolus member capturing device 30. Specifically, each annular unit 34 and the adjacent unit 34 are linked to each other through the link portions 35 connecting the connection portions 36 to each other.

These link portions 35 are substantially unchanged even when the embolus member capturing device 30 is expanded. Since the link portions 35 and the connection portions 36 are substantially unchanged even upon expansion of the embolus member capturing device 30, the overall length of the embolus member capturing device 30 is little changed due to the expansion, so that the embolus member capturing device 30 is prevented from becoming extremely short upon the expansion. In other words, the connection portions 36 connecting the expandable elements are not moved in the axial direction even when the embolus member capturing device 30 is expanded. Since the connection portions 36 are linked by the link portions 35 which are parallel to the axis of the embolus member capturing device 30, the overall length of the embolus member capturing device 30 is little reduced due to the expansion.

The link portions 35 are so provided as to link the adjacent annular units 34 to each other at a plurality of locations. The number of the link portions 35 provided between two adjacent annular units 34 is preferably two or three, particularly two. Furthermore, the link portions 35 are so arranged that each link portion 35 is not in continuity with the link portion 35 adjacent thereto in the axial direction; besides, each link portion 35 is arranged in a direction (angle) different from that of the adjacent link portion 35.

In addition, an outside portion 33b of each of the polygonal filamentous bodies 33 located at both ends of the embolus member capturing device 30 is formed in a semi-elliptic shape. This makes it possible to obtain sufficient expansive forces at the end portions, and to reduce the damage to the inside wall of the blood vessel in which the embolus member capturing device 30 is left to indwell as well as the damage to the expansion body. Besides, all the polygonal filamentous bodies 33 are curved in the circular circumferential direction so that the whole part thereof is substantially equally spaced from the center axis of the embolus member capturing device 30.

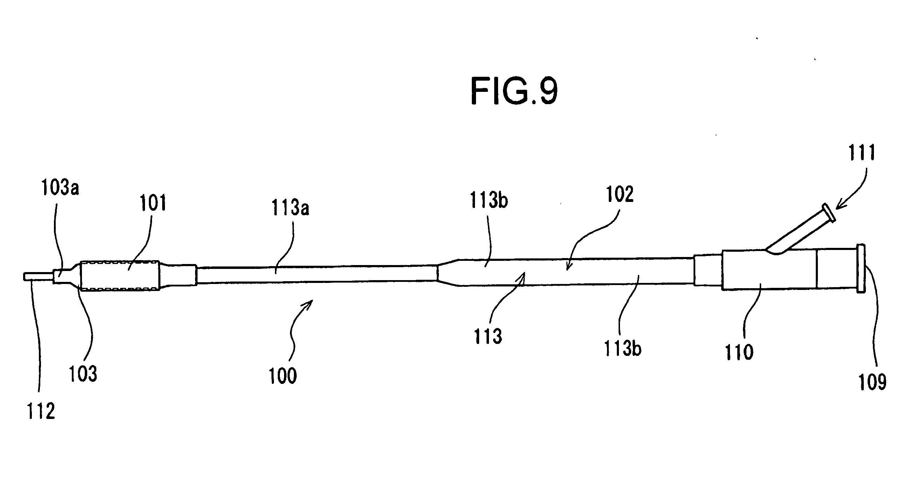

The length of one annular unit 34 in the embolus member capturing device 30, or the length in the axial direction of one polygonal filamentous body 33, is preferably in the range of about 1.5 to 4.0 mm, more preferably 2.0 to 3.0 mm. The number of the annular units 34 in the embolus member capturing device 30 is preferably in the range of three to ten, more preferably three to eight The material thickness of the annular units 34 in a central portion of the embolus member capturing device 30 is preferably in the range of about 0.01 to 0.12 mm, more preferably 0.03 to 0.10 mm. The material thickness of the embolus member capturing device 30 is preferably in the range of about 0.01 to 0.12 mm. In the embolus member capturing device 30, the material thickness of the one end portion 30a is preferably smaller than that of the other end portion 30c. At the time of inserting into a blood vessel an embolus member capturing device leaving implement (see Fig. 9) having the embolus member capturing device mounted to an expansion body 103, the one end portion 30a is flexible and easy to insert into the blood vessel, since the material thickness of the one end portion 30a is smaller than that of the other end portion 30c. It is more preferable that the material thicknesses of the one end portion 30a and the intermediate portion 30b are smaller than the material thickness of the other end portion 30c. The above-mentioned relationship between the material thicknesses is preferably in conformity with the configurations in the embolus member capturing devices 10, 40, 60 described above and described later. The diameter of the embolus member capturing device 30 at the time of molding (before compression) is preferably in the range of about 1.5 to 3.5 mm, more preferably 2.0 to 3.0 mm.

Incidentally, the embolus member capturing device 30 in the above-described possible construction is of the type in which the adjacent annular units 34 are linked to each other by two link portions 35. Therefore, the link portions 35 are located at positions opposite to each other, with the center axis of the embolus member capturing device 30 therebetween. This configuration in this possible construction is not limitative; for example, three or four link portions 35 (or, two to four link portions 35, if the case of the two link portions 35 in the above-described possible construction is included) may be provided between the adjacent annular units 34. In such cases, the link portions 35 are arranged at substantially equal angular intervals around the center axis of the embolus member capturing device 30. Namely, where three link portions 35 are provided between the adjacent annular units 34, the link portions 35 are arranged at an angular interval of about 120 degrees.

The embolus member capturing device may be in the form as shown in Figs. 5 and 6. Fig. 5 is a front view of a further possible construction of the embolus member capturing device. Fig. 6 is a development of the embolus member capturing device shown in Fig. 5.

The embolus member capturing device 40 according to this possible construction is formed to be a tubular body, has a diameter suitable for insertion thereof into a blood vessel, and is expandable when a radially dilating force is exerted from the inside of the tubular body. The embolus member capturing device 40 is composed of annular units 44 each of which is composed of a first wavy annular body 42a formed in an annular shape from a wavy element having a multiplicity of bent portions 45a, a second wavy annular body 42b formed in an annular shape from a wavy element having filamentous bent portions 45a and disposed in the axial direction of the embolus member capturing device 40 so that mount portions thereof are located close to valley portions of the first wavy annular body 42a, and a plurality of connection portions 46 for connection between the valley portions of the first wavy annular body 42a and the mount portions of the second wavy annular body 42b, wherein a plurality of the annular units 44 are arrayed in the axial direction of the embolus member capturing device 40, and the embolus member capturing device 40 includes link portions 47 for linking the adjacent annular units 44 to each other at connection portion forming sites. Furthermore, the link portions 47 are provided between the adjacent annular units 44 in plurality and at opposite positions or at substantially equal angular intervals around the center axis of the embolus member capturing device 40.

The embolus member capturing device 40 is a so-called balloon expandable embolus member capturing device.

The annular units 44 located in one end portion 40a of the embolus member capturing device 40 are provided with extension restrictive portions 48 for restricting extension thereof when a radially dilating force is exerted thereon. Therefore, the one end portion 40a of the embolus member capturing device 40 is slightly enlarged in outside diameter or is substantially unchanged in outside diameter when a radially dilating force is exerted from the inside of the tubular body. On the other hand, the annular units 44 located in the other end portion 40b of the embolus member capturing device 40 are extended when a radially dilating force is exerted thereon, resulting in that the other end portion 40b is enlarged in outside diameter. The annular unit 44 located in an intermediate portion 40c of the embolus member capturing device 40 is not provided with any extension restrictive portion, and is extensible when a radially dilating force is exerted thereon; however, since it is linked to the annular unit 44 constituting the one end portion 40a through the link portions 47, the expansion thereof is restricted, so that the increase in the outside diameter of the intermediate portion 40c is smaller than that of the other end portion 40b. As a result, upon expansion, the outside diameter of the intermediate portion 40c of the embolus member capturing device 40 lies between the outside diameter of the one end portion 40a and the outside diameter of the other end portion 40b. Upon expansion of the embolus member capturing device 40, either the intermediate portion 40c or the one end portion 40a forms an embolus member capturing portion.

In the embolus member capturing device 40 according to this possible construction, the extension restrictive portions 48 are so provided as to link the adjacent connection portions 46 in the annular unit 44 to each other. However, this configuration is not limitative. For example, the extension restrictive portions 48 may be so provided as to link inclined portions of the wavy annular body 42a in the annular unit 44 to each other, or may be so provided as to link inclined portions of the wavy annular body 42a in the annular unit 44 to inclined portions of the opposed wavy annular body 42b; further, the extension restrictive portions 48 may be so provided as to link apexes of the wavy annular body 42a in the annular unit 44 to apexes of the opposed wavy annular body 42b. Though the extension restrictive portions 48 may be formed in a rectilinear shape, it is preferable for the extension restrictive portions 48 to be curved or bent so as to allow some expansion of the one end portion 40a. The curved or bent shape is not limited to the zigzag shape as shown in Figs. 5 and 6, and a gradual arcuate shape, a wedge-like shape or the like may be adopted.

As shown in Figs. 5 and 6, the plurality of annular units 44 are arrayed substantially rectilinearly in the axial direction of the embolus member capturing device 40, and the embolus member capturing device 40 includes the link portions 47 for linking the connection portions 46 of the wavy elements (the wavy annular bodies 42b and 42a) of the adjacent annular units 44 to each other. In other words, the embolus member capturing device 40 is a tubular body constituted by linking a multiplicity of the annular units 44 through the link portions 47.

As shown in Fig. 5 and in Fig. 6, which is a development of Fig. 5, each of the annular units 44 in the embolus member capturing device 40 is constituted of an endless wavy bodies which each have six mounts and six valleys at nearly equal pitches and are each in an annular continuous shape. Incidentally, the number of the mounts (or valleys) in the annular unit is preferably in the range of four to seven. The second wavy annular body 42b is so disposed in the axial direction that the mount portions thereof are located close to the valley portions of the first wavy annular body 42a, and the valley portions of the first wavy annular body 42a and the mount portions of the second wavy annular body 42b are connected to each other through a plurality of the short connection portions 46, to constitute one annular unit 44. In this possible construction, all the valley portions of the first wavy annular body 42a and all the mount portions of the second wavy annular body 42b are connected to each other through the connection portions 46, and each annular unit 44 include six (equal to the number of the mounts or valleys in the annular unit) connection portions 46.

A plurality (ten, in this possible construction) of the annular units 44 configured as above are arrayed in the axial direction of the embolus member capturing device 40, and include the link portions 47 for linking the adjacent annular units 44, whereby the tube-formed embolus member capturing device 40 is formed. The link portions 47 are so provided as to link the adjacent annular units 44 at a plurality of locations. The number of the link portions 47 provided between the adjacent annular units 44 is preferably in the range of two to four. Further, the link portions 47 are so arranged that none of them is continuous with the adjacent one. In this embolus member capturing device 40, two link portions 47 are provided between the adjacent annular units 44. Namely, the adjacent annular units 44 are linked to each other at two locations.

At least one of the plurality of connection portions 46 in each annular unit 44 is a united portion 44a at which the valley portion of the first wavy annular body 42a and the mount portion of the second wavy annular body 42b are united. On the other hand, the other connection portions 46 are thin line form connection portions. At least one of the plurality of link portions 47 provided between the adjacent annular units 44 links the united portions 44a of the adjacent annular units 44 to each other. On the other hand, the other link portions 47 each link the portions forming the thin line form connection portions 46 of the adjacent annular units 44 to each other. Thus, in the embolus member capturing device 40 in this possible construction, the adjacent annular units 44 are linked to each other via their united portions 44a, at each of which the valley portion of the first wavy annular body 42a and the mount portion of the second wavy annular body 42b are united, so that the adjacent annular units 44 are linked to each other with a sufficient link strength. Furthermore, the adjacent annular units 44 include the other (second) link portions 47 for linking the portions forming the thin line connection portions 46 to each other. Therefore, extension of the embolus member capturing device 40 after left to indwell in a living body can be restrained. Simultaneously, curving of the embolus member capturing device 40 is not hindered, since the linking through the other (second) link portions 47 is not a linking via the united portions 44a.

The plurality of link portions 47 for linkage between the annular units 44 are inclined at predetermined angles against the center axis of the embolus member capturing device 40. In other words, in the condition where the embolus member capturing device 40 is developed by cutting it in the longitudinal direction in parallel to the center axis thereof, the link portions 47 are inclined at predetermined angles against the longitudinal direction of the embolus member capturing device 40. Further, in the embolus member capturing device 40 in this possible construction shown in the figures, the link portions 47 for linkage between the two same adjacent annular units 44 are inclined to the same direction and at nearly equal angles. Besides, in the embolus member capturing device 40 in this possible construction, the link portions, which are adjacent to each other in the axial direction of the embolus member capturing device 40, are inclined to the different direction. Furthermore, the link portions 47 are so arranged that none of them is continuous with the adjacent one.

In this embolus member capturing device 40, further, it is preferable that the apex portions of bent portions 45a of the wavy annular body are located in the spaces formed between the adjacent bent portions 45a of the adjacent annular unit This configuration ensures that the embolus member capturing device 40 has the wavy annular bodies partly overlapping each other, as viewed in the axial direction of the embolus member capturing device 40. Even if each of the component elements is shortened in the axial direction of the embolus member capturing device 40 when the embolus member capturing device 40 is expanded, the increase of the gaps in the side surface of the embolus member capturing device 40 will be small, so that a constricted portion of a blood vessel can be expanded more securely, and the diseased site can be pressed without gaps.

The length of each of the wavy annular bodies 42a, 42b in the embolus member capturing device 40 is preferably in the range of about 0.7 to 2.0 mm, and the length of each of the annular units 44 is preferably in the range of about 1.5 to 4.0 mm, more preferably 2.0 to 3.0 mm. The number of the mounts (or the number of the valleys) in each of the wavy annular bodies 42a, 42b is preferably in the range of four to eight, more preferably five to seven. The number of the annular units 44 in the embolus member capturing device 40 is preferably in the range of three to twenty.

Incidentally, the embolus member capturing device 40 in the above-described possible construction is of the type in which the adjacent annular units 44 are linked to each other through two link portions 47. Therefore, the link portions are arranged at positions opposite to each other, with the center axis of the embolus member capturing device 40 therebetween. However, this configuration is not limitative; three or four (or, two to four, if the case of the two link portions 47 as above-described is included) link portions 47 may be provided between the adjacent annular units 44.

The embolus member capturing device may be in the form as shown in Figs. 7 and 8. Fig. 7 is a front view of yet another possible construction of the embolus member capturing device. Fig. 8 is a development of the embolus member capturing device shown in Fig. 7.

As shown in Figs. 7 and 8, in the embolus member capturing device 60 according to this embodiment, a plurality of wavy annular bodies 62 are so arrayed that mount portions or valley portions of the wavy annular bodies 62 adjacent to each other in the axial direction are aligned substantially rectilinearly. Besides, connection portions 64 are provided for connection between the mount portions or valley portions of the adjacent wavy annular bodies 62.

In other words, the embolus member capturing device 60 is composed of a plurality of annular bodies 62 each composed of a wavy (zigzag) and annularly continuous (endless) filamentous body for the role of maintaining expansion, wherein the annular bodies 62 are connected by the connection portions (connectors) 64 so that the adjacent annular bodies 62 will not separate from each other.

This embolus member capturing device 60 is a so-called balloon expandable embolus member capturing device.

The wavy annular bodies 62 located in one end portion 60a of the embolus member capturing device 60 are provided with extension restrictive portions 68 for restricting extension thereof when a radially dilating force is exerted thereon. Therefore, the one end portion 60a of the embolus member capturing device 60 is slightly enlarged in outside diameter or substantially unchanged in outside diameter when a radially dilating force is exerted from the inside of the tubular body. On the other hand, the wavy annular bodies 62 located in the other end portion 60b of the embolus member capturing device 60 are extended when a radially dilating force is exerted thereon, resulting in that the other end portion 60b is enlarged in outside diameter. The wavy annular body 62 located in an intermediate portion 60c of the embolus member capturing device 60 is not provided with any extension restrictive portion, and is extensible when a radially dilating force is exerted thereon; however, since it is linked to the wavy annular body 62 constituting the one end portion 60a through the connection portions 64, the expansion thereof is restrained, and the increase of the outside diameter of the intermediate portion 60c upon expansion is smaller than that of the other end portion 60b. As a result, upon expansion of the embolus member capturing device 60, the outside diameter of the intermediate portion 60c is between the outside diameter of the one end portion 60a and the outside diameter of the other end portion 60b. Upon expansion of the embolus member capturing device 60, either the intermediate portion 60c or the one end portion 60a forms an embolus member capturing portion.

In the embolus member capturing device 60 in this possible construction, the extension restrictive portions 68 are formed to be substantially orthogonal to the center axis of the embolus member capturing device 60, and are in the state of linking a plurality of individual waves formed in each wavy annular body 62 so that the individual waves will not be extended. Therefore, the wavy annular body 62 is extensible between the adjacent waves which are not linked to each other by the extension restrictive portion 68. Incidentally, the extension restrictive portion may be annularly continuous so as to link all the adjacent pairs of the waves. Besides, though the figures show the extension restrictive portion 68 as being provided between intermediate portions of the waves, the extension restrictive portion 68 may be provided between bottom portions of the waves.

In addition, though the extension restrictive portions 68 may be in a rectilinear shape, they may be curved or bent so as to allow some expansion of the one end portion 60a. The curved or bent shape may be a zigzag shape as sown in Figs. 5 and 6, and a gradual arcuate shape, a wedge-like shape or the like may be adopted.

The diameter (D1) of the embolus member capturing devices 10, 30, 40, 60 in a non-expanded state is preferably 3.5 mm or below, more preferably in the range of 1.5 to 2.8 mm. On the other hand, the diameter (D2) of the embolus member capturing devices in an expanded state is preferably in the range of 2.0 to 6.0 mm. Besides, the overall length of the embolus member capturing devices is preferably 30 mm or below, more preferably in the range of 8 to 15 mm.

The material for forming the embolus member capturing devices 10, 30, 40, 60 is preferably a material having a certain degree of bio-compatibility. Examples of the material include stainless steels, tantalum or tantalum alloys, platinum or platinum alloys, gold or gold alloys, and cobalt based alloys. Besides, a blank formed into the shape of the embolus member capturing device may be plated with a noble metal (gold, platinum). Among stainless steels, preferred is SUS316L having the highest corrosion resistance.

Furthermore, the embolus member capturing device formed into the final shape is preferably annealed. By the annealing, the embolus member capturing device as a whole is enhanced in flexibility and plasticity, promising good indwelling performance of the embolus member capturing device in bent blood vessels. As compared with the case where annealing is not applied, the annealing reduces the restoring force of the embolus member capturing device after expansion for restoring to its shape before expansion, particularly, the restoring force for returning to a rectilinear shape which is developed when the embolus member capturing device is expanded in a bent blood vessel site, whereby the physical stimulus exerted on the inside wall of the bent blood vessel is reduced, and a cause of restenosis can be reduced. For preventing an oxide film from being formed on the surface of the embolus member capturing device, the annealing is preferably carried out by heating to a temperature of 900 to 1200 degree C in an inert gas atmosphere (e.g., argon gas), followed by slow cooling.

In addition, the embolus member capturing device is preferably chamfered. The chamfering of the embolus member capturing device may be carried out by chemical polishing, electropolishing or mechanical polishing, after the blank is processed to the final shape of the embolus member capturing device. The chemical polishing is preferably carried out by immersion in a stainless steel chemical polishing liquid. The stainless steel chemical polishing liquid may be any liquid that can dissolve stainless steel; a preferable example being a liquid which contains a hydrochloric acid-nitric acid mixture as a basic component and to which an organic sulfur compound and a surfactant have been added for control of dissolving rate, for smoothing, and for imparting a lustrous property.

Furthermore, each of all the embolus member capturing devices in the above-described possible constructions is preferably provided with a contrast marker or markers, as in the case of the embolus member capturing device 30 shown in Figs. 3 and 4. The contrast marker is preferably provided at an end portion of the embolus member capturing device. It is particularly preferable to provide the contrast markers at both end portions of the embolus member capturing device. Specifically, as in the case of the embolus member capturing device 30 shown in Fig. 3 and Fig. 4 (a development of the embolus member capturing device at the time of production), it is preferable to provide a plurality of contrast markers 39 in the axial direction on one end side (specifically, on the distal end side) and to provide contrast markers 39 also on the other end side (specifically, on the proximal end side). This ensures easy confirmation of the positions of the end portions of the embolus member capturing device.

In the case shown in Fig. 3, the contrast markers are formed by plugging up small openings in the embolus member capturing device with contrast markers, whereby the contrast markers are fixed to the embolus member capturing device. The contrast marker is preferably attached, for example, by a method in which a disc form member of a contrast material slightly smaller than a small opening formed in the embolus member capturing device is placed in the small opening, and then the disc form member is caulked by pressing from both sides thereof. The contrast marker is not limited to the above-mentioned one, and may be any one. Examples of the contrast marker include those formed by coating an outside surface of the embolus member capturing device with a contrast material, those formed by winding a wire of a contrast material around the embolus member capturing device, and those formed by attaching a ring-shaped member of a contrast material to the embolus member capturing device. Preferable examples of the material for forming the contrast markers include gold, platinum, tungsten, alloys thereof, and silver-palladium alloys. Incidentally, contrast markers may be any of those for radiography, those for sonography, and those for NMR imaging.

Furthermore, it is preferable that a part or the whole surface of each of the embolus member capturing devices 10, 30, 40, 60 is coated with a highly thrombus-generating material or a fibrous member formed of a highly thrombus-generating material is attached to a part or the whole surface of each of the embolus member capturing devices 10, 30, 40, 60.

Examples of the highly thrombus-generating material include silk yarn, polyesters (e.g., Dacron), nylon, and PET. It is preferable to coat the inside surface of the embolus member capturing device with the highly thrombus-generating material. The inside surface of the embolus member capturing device may be entirely coated with the material, the inside surfaces of the one end portion and the intermediate portion of the embolus member capturing device may be coated with the material, or only the inside surface of the intermediate portion may be coated with the material. Alternatively, it is preferable to attach a fibrous member of the above-mentioned material is attached to the inside surface of the embolus member capturing device. A plurality of the fibrous members may be attached to the whole part of the inside surface of the embolus member capturing device; alternatively, the fibrous members may be attached to the inside surfaces of the one end portion and the intermediate portion, or the fibrous member is attached only to the inside surface of the intermediate portion. Besides, the fibrous members may be attached to the outside surfaces of the one end portion and the intermediate portion, or the fibrous member may be attached only to the outside surface of the intermediate portion. Examples of the method for the attachment include a method in which the fibrous member is adhered to the embolus member capturing device by an adhesive or the like, and a method in which a cut is formed in a part of the embolus member capturing device by laser or the like and the fibrous member is clamped in the cut

Besides, in each of the embolus member capturing devices 10, 30, 40, 60, the second outside diameter on the other end side upon expansion can be regulated by the radially dilating force exerted from the inside of the tubular body. In other words, in each of these embolus member capturing devices, the expanded diameter on the other end side on a configuration basis is not particularly restricted. Therefore, it can be regulated by the radially dilating force exerted from the inside of the tubular body, i.e., the expansive force of the expansion body to which the embolus member capturing device is mounted.

In the next place, an embolus member capturing device leaving implement (in other words, an embolus member capturing device delivery instrument) possible constructions will be described using some thereof.

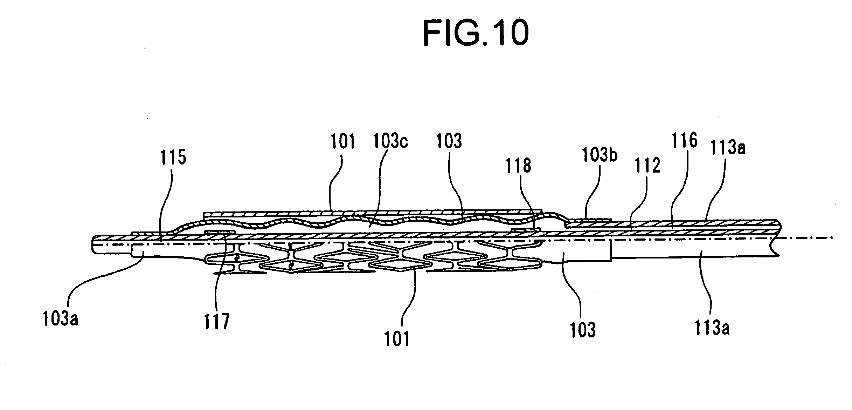

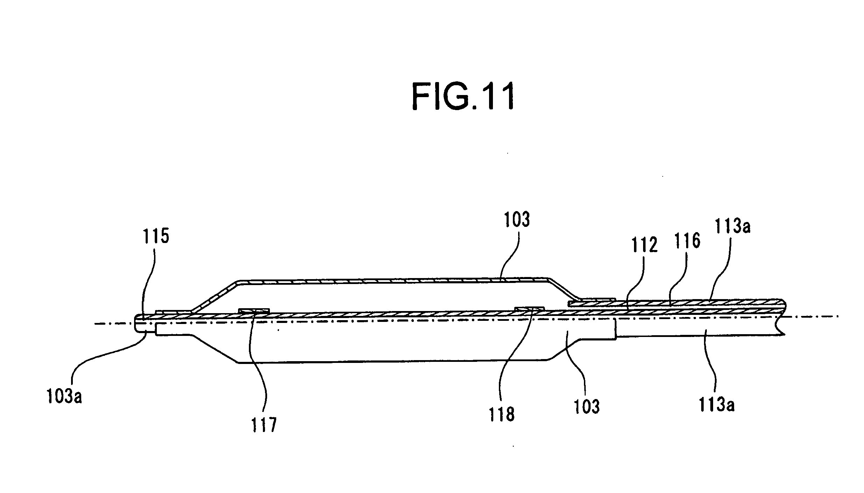

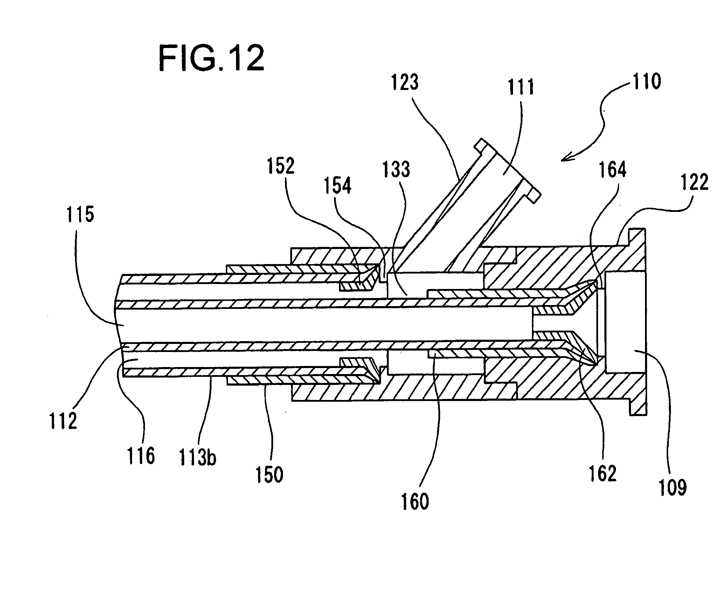

Fig. 9 is a front view of one embodiment of the embolus member capturing device leaving implement which does not form a part of the present invention. Fig. 10 is a partly broken enlarged view of a distal end portion of the embolus member capturing device leaving implement shown in Fig. 9. Fig. 11 is a view showing an expanded state of an expansion body of the embolus member capturing device leaving implement in the condition where an embolus member capturing device is not mounted. Fig. 12 is a partly broken enlarged view of a proximal end portion of the embolus member capturing device leaving implement shown in Fig. 9.

The embolus member capturing device leaving implement 100 in this possible construction includes a catheter 102 having an expandable and foldable expansion body 103 provided in the vicinity of a distal end portion thereof, and an embolus member capturing device 101 mounted to the catheter 102 so as to envelope the expansion body 103.

As the embolus member capturing device 101, there is used an embolus member capturing device as described above. Specifically, any one of the embolus member capturing devices 10, 30, 40, 60 may be used.

The embolus member capturing device leaving implement 100 includes the catheter 102 including the expansion body 103, and the embolus member capturing device 101 mounted to the catheter 102 so as to envelope the expansion body 103.

As shown in Figs. 9 to 11, in the embolus member capturing device leaving implement 100 in this possible construction, the catheter 102 includes a guide wire lumen 115 of which one end is opened at the distal end of the catheter 102 and the other end is opened at a proximal end portion of the catheter 102.

The catheter 102 includes an inner tube 112, an outer tube 113, and a branched hub 110. As shown in Figs. 9 and 11, the inner tube 112 is a tube body including the guide wire lumen 115 for passing a guide wire therein. The inner tube 112 is passed through the inside of the outer tube 113, and its distal end portion protrudes from the outer tube 113. The outside surface of the inner tube 112 and the inside surface of the outer tube 113 form an expansion body expanding lumen 116, which has a sufficient inside volume. The outer tube 113 is a tube body for passing the inner tube 112 therein, and its distal end is located slightly on the proximal side relative to the distal end of the inner tube 112.

In the embolus member capturing device leaving implement 100 according to this possible construction, the outer tube 113 is composed of a distal side outer tube 113a and a main body side outer tube 113b, which are joined to each other. The distal side outer tube 113a is reduced in diameter in a taper form at its portion on the distal side relative to the joint portion for joining to the main body side outer tube 113b, and has a small diameter on the distal side of the tapered portion.

The outside diameter of the distal side outer tube 113a at the small diameter portion is in the range of 0.50 to 1.5 mm, preferably 0.60 to 1.1 mm. The outside diameter of a proximal end portion of the distal side outer tube 113a and the main body side outer tube 113b is in the range of 0.75 to 1.5 mm, preferably 0.9 to 1.1 mm.

The expansion body 103 has a distal side joint portion 103a and a proximal side joint portion 103b, the distal side joint portion 103a is fixed to a distal end portion (specifically a position slightly on the proximal side relative to the distal end) of the inner tube 112, and the proximal side joint portion 103b is fixed to a distal end portion of the outer tube 113. Besides, the expansion body 103 is communicated with the expansion body expanding lumen 116 in the vicinity of a proximal end portion thereof.

The material for forming the inner tube 112 and the outer tube 113 is preferably a material which has a certain degree of flexibility. Examples of the material include thermoplastic resins such as polyolefins (e.g., polyethylene, polypropylene, ethylene-propylene copolymer, ethylene-vinyl acetate copolymer, etc.), polyvinyl chloride, polyamide elastomers, polyurethane, etc., silicone rubbers, and latex rubbers, among which preferred are the thermoplastic resins, and more preferred are the polyolefins.

The expansion body 103 is foldable, as shown in Fig. 11; in its non-expanded state, the expansion body 103 can be folded onto the outer circumference of the inner tube 112. As shown in Fig. 11, the expansion body 103 has an expandable portion which is a tubular portion (preferably, a hollow cylindrical portion) having a substantially equal diameter so that it can expand the embolus member capturing device 101 mounted thereto. The hollow cylindrical portion may not necessarily be perfectly hollow cylindrical, and may be in a polygonal columnar shape. In the expansion body 103, as described above, the distal side joint portion 103a is attached liquid-tight to the inner tube 112 and the proximal side joint portion 103b is attached liquid-tight to the distal end of the outer tube 113, by an adhesive or by fusing or the like. Besides, this expansion body 103 is tapered between the expandable portion and each of the joint portions 103a, 103b. The expansion body 103 has a taper-shape expandable portion formed between the expandable portion and the distal joint portion 103.

The expansion body 103 forms an expansion space 103c between the inside surface of the expansion body 103 and the outside surface of the inner tube 112. The expansion space 103c is communicated, at its proximal end portion, with the expanding lumen 116 over the entire circumference thereof. Thus, the proximal end of the expansion body 103 is communicated with the expanding lumen 116 having a comparatively large inside volume, which promises assured injection of an expanding fluid into the expansion body 103 via the expanding lumen 116.

The material for forming the expansion body 103 is preferably a material which has a certain degree of flexibility. Examples of the material include thermoplastic resins such as polyolefins (e.g., polyethylene, polypropylene, ethylene-propylene copolymer, ethylene-vinyl acetate copolymer, cross-linked ethylene-vinyl acetate copolymer, etc.), polyvinyl chloride, polyamide elastomers, polyurethane, polyesters (e.g., polyethylene terephthalate), polyarylene sulfides (e.g., polyphenylene sulfide), etc., silicone rubbers, and latex rubbers. Among these materials, those that can be oriented are particularly preferable. The expansion body 103 is preferably formed of a biaxially oriented material being high in strength and expansive force.

The expansion body 103 has such a size that the outside diameter of the hollow cylindrical portion (expandable portion) upon expansion is in the range of 1.5 to 6.5 mm, preferably 2.0 to 6.0 mm, and the length thereof is in the range of 5 to 35 mm, preferably 8 to 15 mm.

The catheter 102 is provided with two contrast members 117 and 118 fixed to the outside surface of the catheter 102 at positions corresponding to both end portions of the embolus member capturing device 101 to be mounted. Each of the contrast members 117, 118 is preferably a ring-shaped one, one formed by winding a filamentous material in a coil shape, or the like which has a predetermined length. Examples of the preferable material for forming the contrast members 117, 118 include gold, platinum, tungsten, alloys thereof, and silver-palladium alloys.

In the embolus member capturing device leaving implement 100 according to this possible construction, as shown in Fig. 12, the branch hum 110 is fixed to the proximal end.

The branched hub 110 is composed of: an inner tube hub 122 which has a guide wire introduction port 109 communicated with the guide wire lumen 115 so as to form a guide wire port and which is attached to the inner tube 112; and an outer tube hub 123 which is communicated with the expansion body expanding lumen 116, has an injection port 111, and is attached to the outer tube 113. The outer tube hub 123 and the inner tube hub 122 are attached to each other. Examples of the preferable material for forming the branched hub 110 include thermoplastic resins such as polycarbonates, polyamides, polysulfones, polyarylates, methacrylate-butylene-styrene copolymer, etc.

In this possible construction, a bending preventive tube 150 is provided at a proximal end portion of the outer tube 113. The bending preventive tube 150 is formed from a thermally shrinkable material so that the inside diameter thereof upon thermal shrinkage is slightly smaller than the outside diameter of the outer tube 113. The thus formed tube 150 is mounted in position by fitting it over the proximal end portion of the outer tube 113 and then heating it (for example, by blowing a hot air flow thereto). In addition, the bending preventive tube 150 is fixed to the outer tube hub 123 by a stop pin 152. The fixation is carried out by a method in which the stop pin 152 having an outside diameter nearly equal to the inside diameter of the outer tube 113 at other portions than a proximal end portion thereof and having a radially enlarged proximal end portion is inserted into the proximal end of the outer tube 113, the outer tube 113 is inserted into the outer tube hub 123 starting from the distal end thereof, and the outer tube 113 is pushed in until a proximal end portion of the stop pin 152 comes beyond a projection 154 provided on the inside surface of the outer tube hub 123. Further, the outer tube hub 123 and the bending preventive tube 150 may be attached to each other by applying an adhesive to their contact surfaces.

In addition, a bending preventive tube 160 is provided at a proximal end portion of the inner tube 112. The tube 160 is formed from a thermally shrinkable material so that the inside diameter thereof upon thermal shrinkage is slightly smaller than the outside diameter of the inner tube 112, and the thermally shrinkable tube 160 can be easily mounted in position by fitting it over the proximal end portion of the inner tube 112 and then heating it (for example, by blowing a hot air flow thereto). The inner tube 112 thus fitted with the bending preventive tube 160 is fixed to the inner tube hub 122. The fixation is carried out by a method in which a stop pin 162 having an outside diameter nearly equal to the inside diameter of the inner tube 112 at other portions than a proximal end portion thereof and having a radially enlarged proximal end portion is inserted into the proximal end of the inner tube 112, then the inner tube 112 is inserted into the inner tube hub 122 starting from the distal end thereof, and the inner tube 112 is pushed in until the proximal end portion of the stop pin 162 comes beyond a projection 164 provided on the inside surface of the inner tube hub 122. Further, the inner tube hub 122 and the bending preventive tube 160 may be attached to each other by applying an adhesive to their contact surfaces.

Examples of the preferable material for forming the outer tube hub 123 and the inner tube hub 122 include thermoplastic resins such as polycarbonates, polyamides, polysulfones, polyarylates, methacrylate-butylene-styrene copolymer, etc.

The inner tube hub 122 and the outer tube hub 123 are fixed to each other. The fixation is conducted by a method in which the inner tube 112 is inserted from the proximal end of the outer tube hub 123 attached to the proximal end portion of the outer tube 113, starting from the distal end of the inner tube 112, and the inner tube hub 122 and the outer tube hub 123 are joined to each other. In this case, when an adhesive is applied to the joint portion between the inner tube hub 122 and the outer tube hub 123, both of the hubs can be attached to each other more assuredly.

Incidentally, the structure of the proximal end of the embolus member capturing device leaving implement 100 is not limited to the above-described structure. For example, a structure may be adopted in which the branched hub 110 is not provided, and tubes having a port member for forming an opening portion at the proximal end thereof are attached liquid-tight to the guide wire lumen 115 and the expansion body expanding lumen 116, respectively.

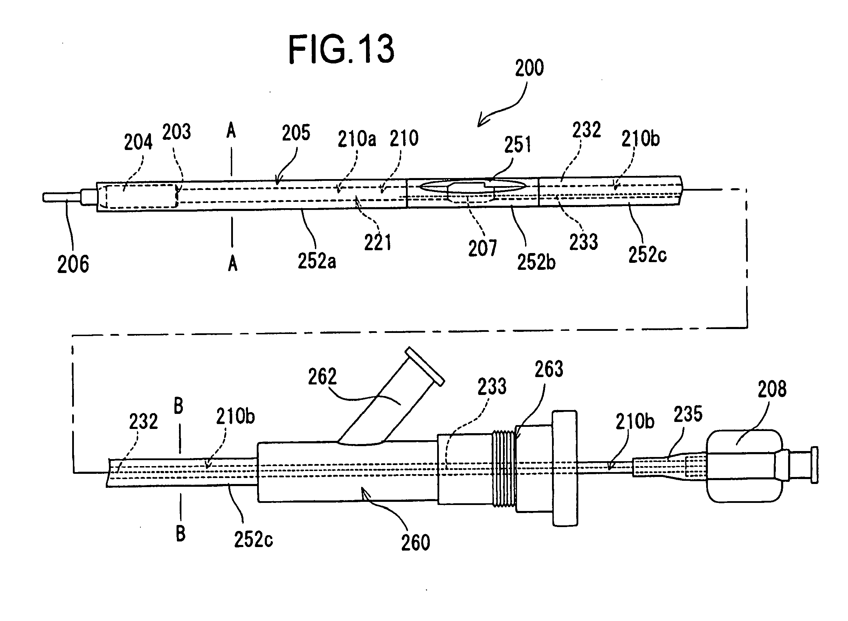

In addition, the embolus member capturing device leaving implement is not limited to the over-the-wire type one such as the above-described embolus member capturing device leaving implement 100, and may be any of those ones which have a guide wire insertion port at an intermediate portion of a catheter (so-called rapid exchange type) as shown in Figs. 13 to 24. An embolus member capturing device leaving implement 200 according to this possible construction includes a sheath 205 through which the catheter 210 with an embolus member capturing device mounted thereto can be passed. Incidentally, a sheath permitting the passage of the catheter therethrough may be provided in the embolus member capturing device leaving implement 100 in the above-described possible construction. In addition, the embolus member capturing device leaving implement 200 in this possible construction may lack the sheath 205.

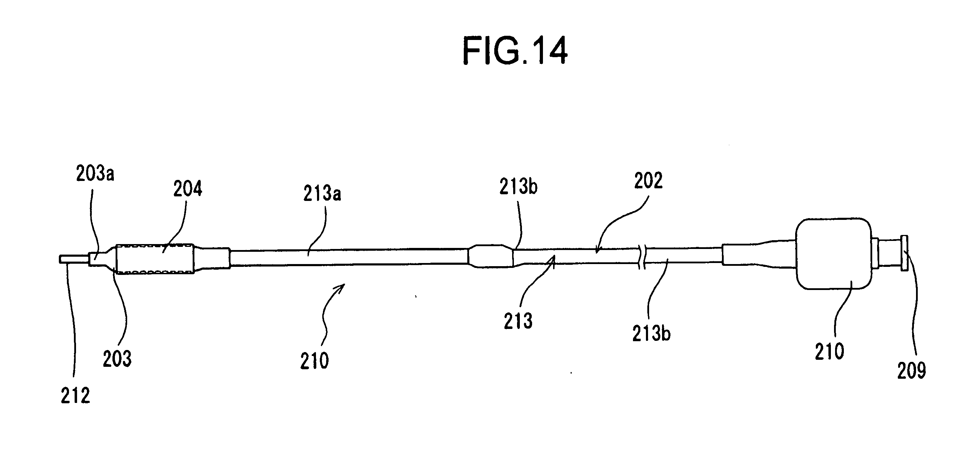

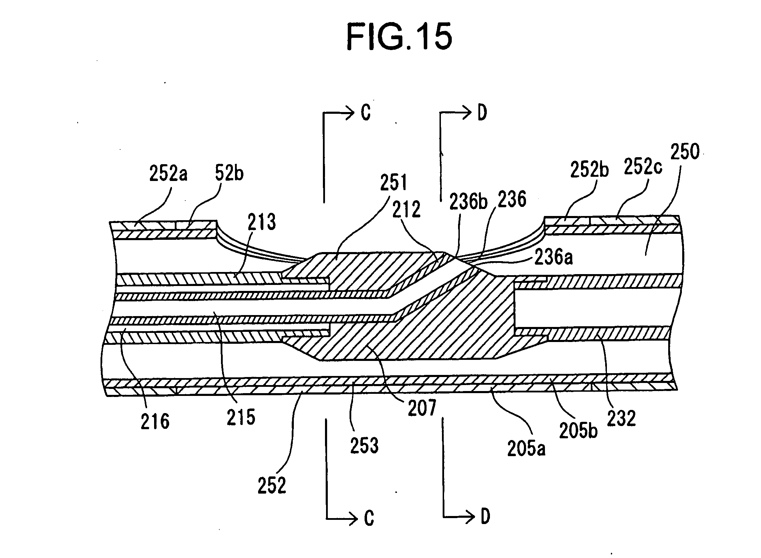

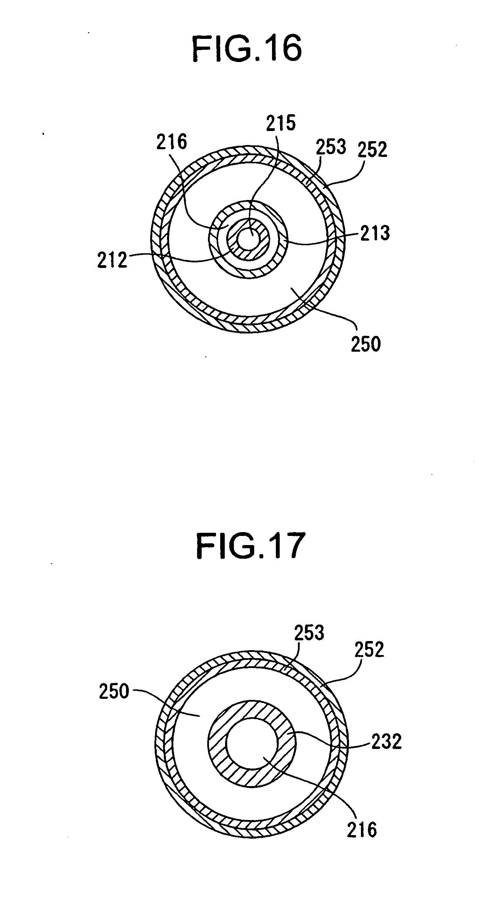

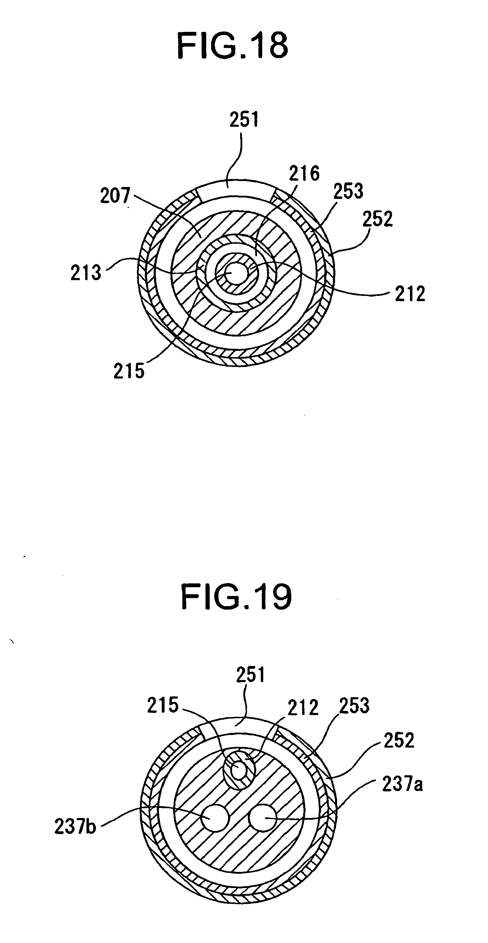

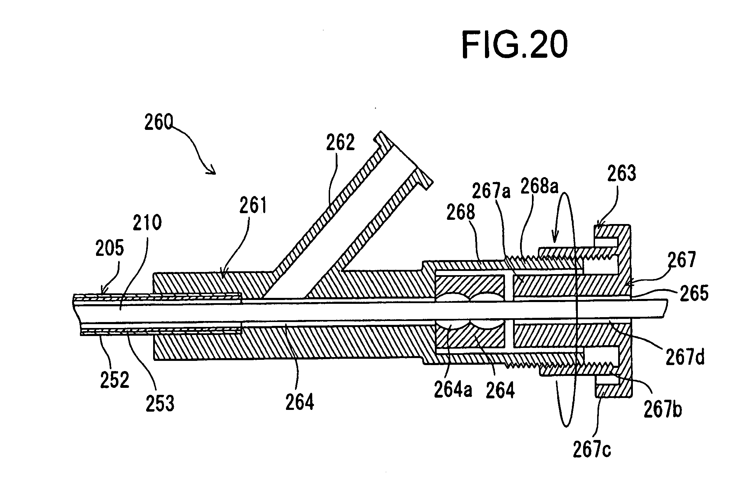

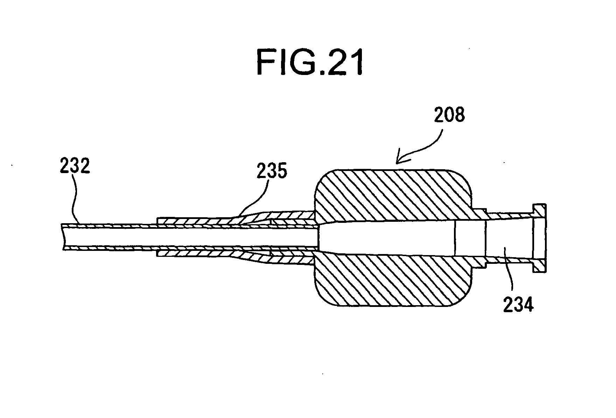

Fig. 13 is a front view of another possible construction of the embolus member capturing device leaving implement, which does not form a part of the present invention. Fig. 14 is a view showing the condition where a sheath is removed from the embolus member capturing device leaving implement shown in Fig. 13. Fig. 15 is an enlarged sectional view of a central joint portion of the embolus member capturing device leaving implement shown in Fig. 13. Fig. 16 is an end view along line A-A of Fig. 13. Fig. 17 is an end view along line B-B of Fig. 13. Fig. 18 is a sectional view along line C-C of Fig. 15. Fig. 19 is a sectional view along line D-D of Fig. 15. Fig. 20 is an enlarged sectional view of a proximal end portion of the embolus member capturing device leaving implement shown in Fig. 13. Fig. 21 is an enlarged sectional view of a proximal end portion of a catheter used for the embolus member capturing device leaving implement shown in Figs. 13 and 14.

The embolus member capturing device leaving implement 200 according to this possible construction includes: an expanding catheter 210 including a tubular shaft main body portion 221, a foldable and expandable expansion body (balloon) 203 provided at a distal end portion of the shaft main body portion 221, an embolus member capturing device 204 so mounted as to envelope the balloon 203 in the folded state and expanded by expansion of the balloon 203, and a guide wire lumen 215 of which one end is opened at the distal end of the shaft main body portion 221 and the other end is opened at an intermediate portion of the shaft main body portion 221; and a sheath 205 having a catheter lumen 250 for slidably containing the expanding catheter 210 therein.

The sheath 205 is provided with an axially extending side hole 251 for inserting a guide wire 100 into the guide wire lumen 215, the side hole 251 being provided at a position in the vicinity of the other end side opening portion of the guide wire lumen 215 of the expanding catheter 210.

The embolus member capturing device leaving implement 200 is of the so-called rapid exchange type, and has the guide wire lumen 215 provided in the inside of the expanding catheter 210 and opened at the distal end and an intermediate portion of the expanding catheter 210 (other end side opening portion 236), and the side hole 251 provided in an intermediate portion of the sheath 205 for insertion of the guide wire, as shown in Figs. 13 to 21. This configuration ensures that, with the embolus member capturing device leaving implement 200, an embolus member capturing device can be operated by inserting the guide wire from an intermediate portion of the sheath 205 into the opening (other end side opening portion 236) provided in the intermediate portion of the catheter 210 via the side hole 251 formed in the sheath 205.

The expanding catheter 210 is composed of a distal side shaft portion 210a and a proximal side shaft portion 210b. The distal side shaft portion 210a and the proximal side shaft portion 210b are joined to each other through a joint connector 207, as shown in Fig. 15. Besides, in the sheath 205, the expanding catheter 210 is slidable from the condition where the distal end of the embolus member capturing device 204 of the expanding catheter 210 is contained in the sheath 205 to the condition where the proximal end of the embolus member capturing device 204 is exposed.

The distal side shaft portion 210a has the same configuration as shown in Fig. 10 (see Fig. 15), and is composed of an inner tube 212 for forming a guide wire lumen 215, a balloon 203 provided at a distal end portion of the inner tube 212, an embolus member capturing device 204 mounted on the outer circumference of the balloon 203, and an outer tube 213 provided on the proximal side relative to the balloon 203 and enveloping the inner tube 212 so as to form a balloon expanding lumen 216 between itself and the outside surface of the inner tube 212. The distal side shaft portion 210a is slidably contained in the catheter lumen 250 of the sheath 205. Besides, a proximal end portion of the distal side shaft portion 210a is joined to a distal end portion of the joint connector 207.

The balloon 203 is the same as that described in the above-described possible construction.

The material for forming the inner tube 212 and the outer tube 213 is preferably a material which has a certain degree of flexibility. Examples of the material include thermoplastic resins such as polyamides, polyesters, polyolefins (inclusive of cross-linked ones and partly cross-linked ones), polyvinyl chloride, polyurethane, etc., silicone rubbers, and latex rubbers, among which preferred are the thermoplastic resins.

In the same manner as in the embolus member capturing device leaving implement 100 in the above-described possible construction, a distal side contrast marker may be fixed to the outside surface of the shaft main body portion 221 (in this possible construction, the inner tube 212) at a position located near the distal end of the inside of an expandable portion 231 of the balloon 203.

As the embolus member capturing device 204 used for the embolus member capturing device leaving implement 200, there is used any of the above-mentioned embolus member capturing devices. Specifically, any one of the embolus member capturing devices 10, 30, 40, 60 may be used.

The proximal side shaft portion 210b is composed of a shaft tube 232, and a hub 208 fixed to the proximal end of the shaft tube 232, as shown in Fig. 21. The proximal side shaft portion 210b is slidably contained in the catheter lumen 250 of the sheath 205. Besides, a distal end portion of the proximal side shaft portion 210b is joined to a proximal end portion of the joint connector 207.

The material for forming the shaft tube 232 is preferably a material which has a certain degree of flexibility. Examples of the material include thermoplastic resins such as polyolefins (e.g., polyethylene, polypropylene, ethylene-propylene copolymer, ethylene-vinyl acetate copolymer, etc.), polyvinyl chloride, polyamide elastomers, polyimides, polyurethane, etc., silicone rubbers, and latex rubbers, among which preferred are the thermoplastic resins. Further, a stainless steel pipe may be used as the material for forming the shaft tube 232.

As shown in Fig. 15, the joint connector 207 includes an inner tube insertion passage which extends in the axial direction from the center of the distal end to a central portion, is curved from the central portion, and reaches the outside surface on the proximal end side. A proximal end portion of the inner tube 212 penetrates through the insertion passage, and the proximal end portion of the inner tube 212 protruding from a side surface of the joint connector 207 forms a guide wire introduction port 236 (other end side opening portion 236). The opening of the other end side opening portion 236 is formed toward an obliquely upward proximal side in Fig. 15. Incidentally, the direction of the opening is not limited to the one in this embodiment; the opening may be formed just upwards in Fig. 15. In addition, the joint connector 207 is provided with balloon expanding fluid conduits 237a and 237b extending from the distal end to the proximal end. A balloon expanding lumen 216 formed between the inner tube 212 and the outer tube 213 and a balloon expanding lumen 216 formed in the shaft tube 232 are communicated with each other through the conduits 237a, 237b.

As shown in Figs. 13, 15 and 20, the sheath 205 is formed in a tubular shape, is provided therein with a catheter lumen 250, and is provided with a side hole 251 in its intermediate portion. Besides, a proximal end portion of the sheath 205 is joined to a distal end portion of the branched hub 260.

The catheter lumen 250 is a passage for slidably containing the expanding catheter 210 therein, as shown in Fig. 15. In other words, the catheter lumen 250 is a passage permitting passage of the expanding catheter 210 therethrough; the distal end of the expanding catheter 210 protrudes beyond the distal end of the catheter lumen 250, namely, beyond the distal end of the sheath 205, and a proximal end portion of the expanding catheter 210 protrudes beyond the proximal end of the catheter lumen 250, namely, beyond the proximal end of the sheath 205.

The side hole 251 is provided for inserting a guide wire (not shown) into the guide wire lumen 215. The guide wire is inserted through the side hole 251 into the other end side opening portion 236, and is introduced into the guide wire lumen 215.

As shown in Figs. 13 and 15, the side hole 251 is formed as an axially extending elliptic opening portion, and is formed at a position located in the vicinity of the other end side opening portion 236 (which is a guide wire insertion port) of the guide wire lumen 215, so as to be communicated with the catheter lumen 250.

In the sheath 205, the expanding catheter 210 is slidable from the condition where the distal end of the embolus member capturing device 204 of the expanding catheter 210 is contained in the sheath 205 to the condition where the proximal end of the embolus member capturing device 204 is exposed. The side hole 251 in the sheath 205 has an axial length comparable to or greater than the length of the embolus member capturing device 204. In addition, when not locked by a catheter lock mechanism 263, the expanding catheter 210 is slidable in the sheath 205 from the condition where the distal end of the embolus member capturing device 204 of the expanding catheter 210 is contained in the sheath 205 to the condition where the proximal end of the embolus member capturing device 204 is exposed.

As shown in Fig. 15, the sheath 205 is composed of a sheath outer layer 252, and a sheath inner layer 253 formed on the inner side of the sheath outer layer 252. The sheath outer layer 252 and the sheath inner layer 253 are united. Incidentally, while the sheath is formed in a two-layer structure composed of the outer layer and the inner layer in this embodiment, the sheath may be formed in a multi-layer structure composed of three or more layers. Further, the sheath may have a monolayer structure.

As shown in Fig. 20, the branched hub 260 is composed of a branched hub main body portion 261, a priming injection port 262 provided at a central portion of the branched hub main body portion 261 so as to branch from the main body portion 261, the catheter lock mechanism 263 provided at a proximal end portion of the branched hub 260 for the purpose of restricting the movement of the expanding catheter 210, and an expanding catheter lumen 264 provided to extend from the distal end to the proximal end of the branched hub 260. The catheter lumen diameter at the proximal end 265 of the branched hub 260 is smaller than that at a distal end portion of the hub 208 so that the distal end portion of the hub 208 cannot move to the distal side from the vicinity of the proximal end of the branched hub 260. Besides, a proximal end portion of the sheath 205 is joined to a distal end portion of the branched hub 260. It is preferable that the proximal end portion of the sheath 205 is fixed at a position spaced from the distal end portion of the branched hub 260 toward the proximal side by 1 to 5 mm.

The lock mechanism 263 is composed of an elastic body 264 for clamping a proximal end portion of the expanding catheter 210 by compression, and an operating body 267 for compressing the elastic body 264. With the lock mechanism 263 thus provided, the expanding catheter 210 is fixed at an arbitrary position relative to the sheath 205. The elastic body 264 is disposed inside a proximal end portion 268 of the branched hub main body portion 261, and the elastic body 264 is provided therein with a lumen 264a which forms a part of the catheter lumen 250. In addition, the inside diameter of the proximal end portion 268 is slightly greater than the outside diameter of the elastic body 264 so that the elastic body 264 can be radially enlarged when compressed by the operating body 267. The lumen 264a of the elastic body 264 is formed in such a shape that two spherical shapes partly overlap each other in the axial direction; thus, the diameter of the lumen 264a is reduced at both ends and a central portion of the lumen 264a. Incidentally, the shape of the lumen 264a is not limited to the shape in this embodiment, and may be any shape that promises accurate locking of the expanding catheter 210.

The operating body 267 is composed of an elastic body pressing portion 267a projecting toward the distal side at a central portion, a meshing portion 267b formed near the outer circumference of the elastic body pressing portion 267a and meshing with the proximal end portion 268a of the proximal end portion 268, and a grip portion 267c formed near the outer circumference of the meshing portion 267b so as to be gripped at the time of rotating the operating body 267. In addition, the elastic body pressing portion 267a is provided therein with a lumen 267d which forms a part of the catheter lumen 250. Besides, a distal side portion of the elastic body pressing portion 267a is contained in the proximal end portion 268 as shown in Fig. 20 so as to compress the elastic body 264 when the operating body 267 is moved toward the distal side.

With this configuration, when the operating body 267 is rotated to mesh to the distal side relative to the branched hub 260, the distal end of the elastic body pressing portion 267a makes contact with the proximal end of the elastic body 264, and when the operating body 267 is further rotated to mesh to the distal side, the elastic body 264 is compressed in the axial direction. As the compression proceeds, the inside diameter of the lumen 264a is decreased. Finally, a proximal end portion of the expanding catheter 210 is fixed by the elastic body 264. Incidentally, unlocking of the lock mechanism 263 is conducted by a rotation in the direction reverse to the above.

In the next place, embolus member capturing device leaving implements according to other possible construction of the present invention will be described below.

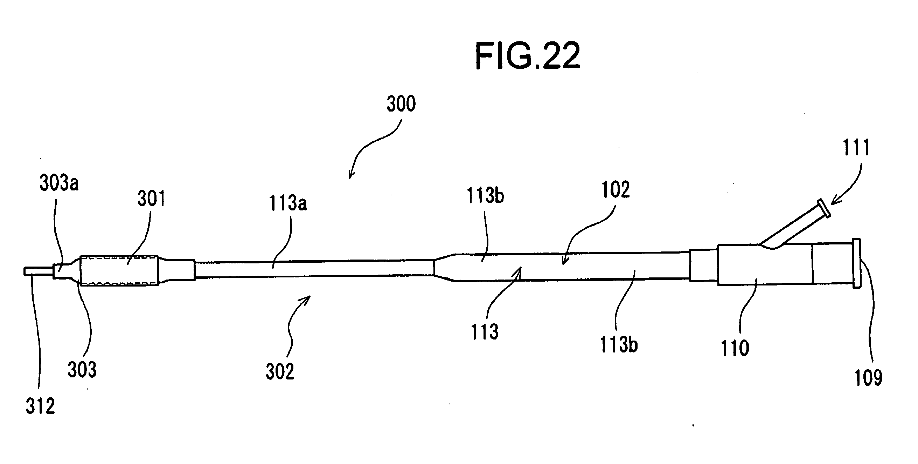

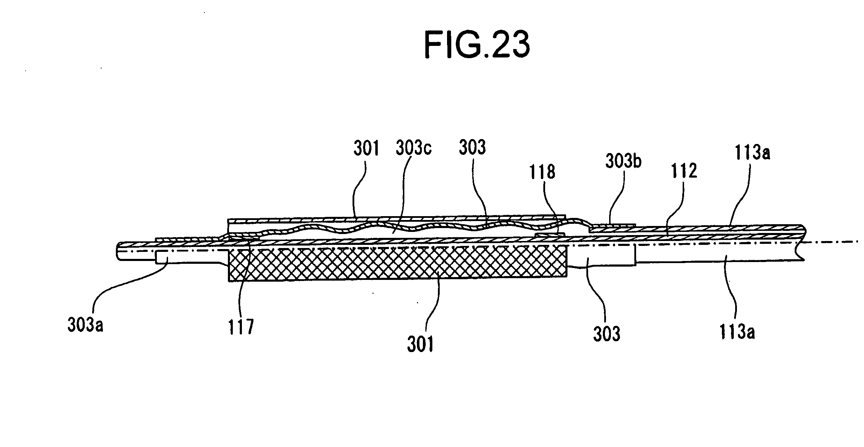

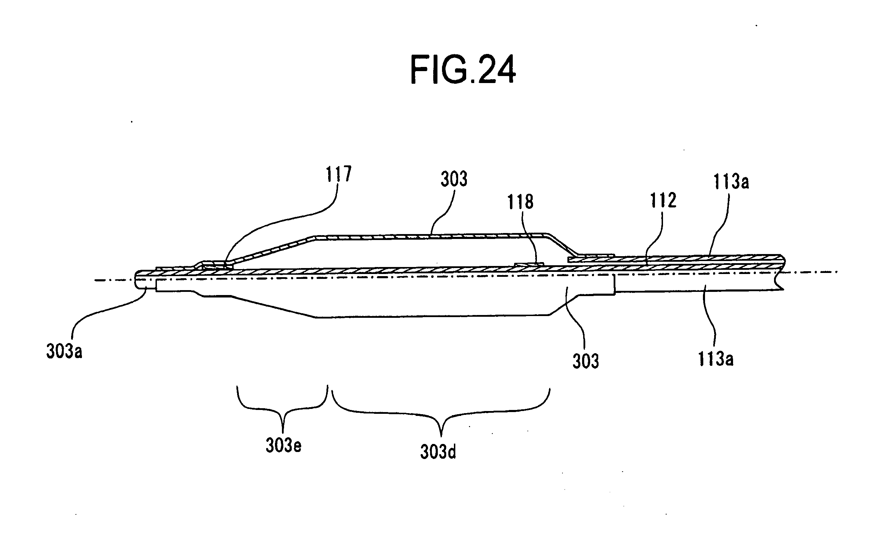

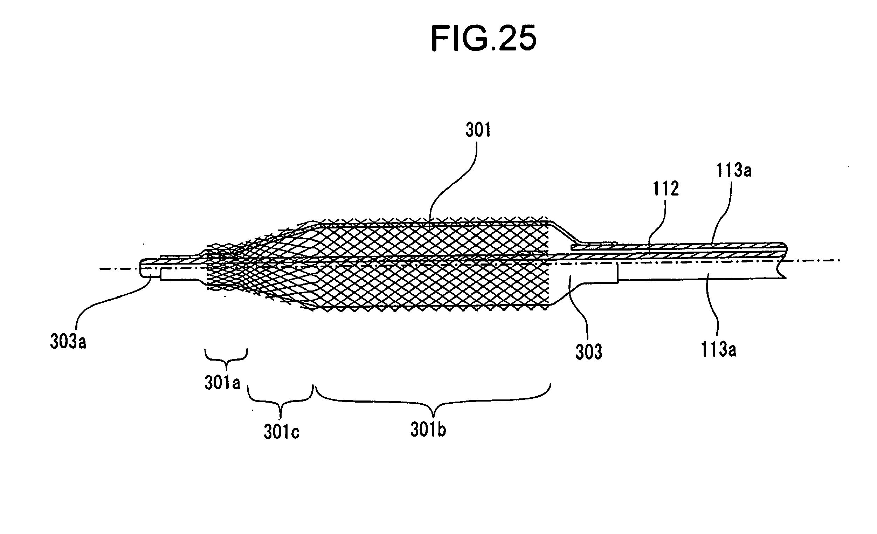

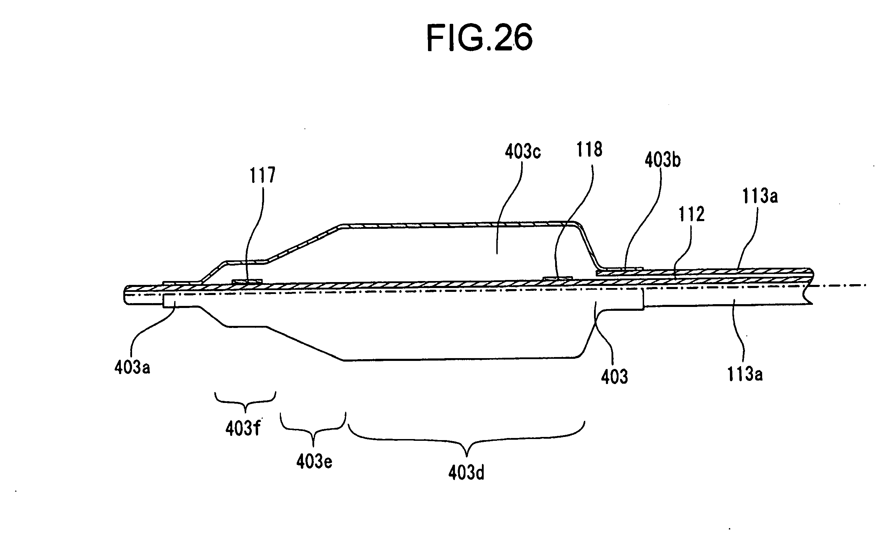

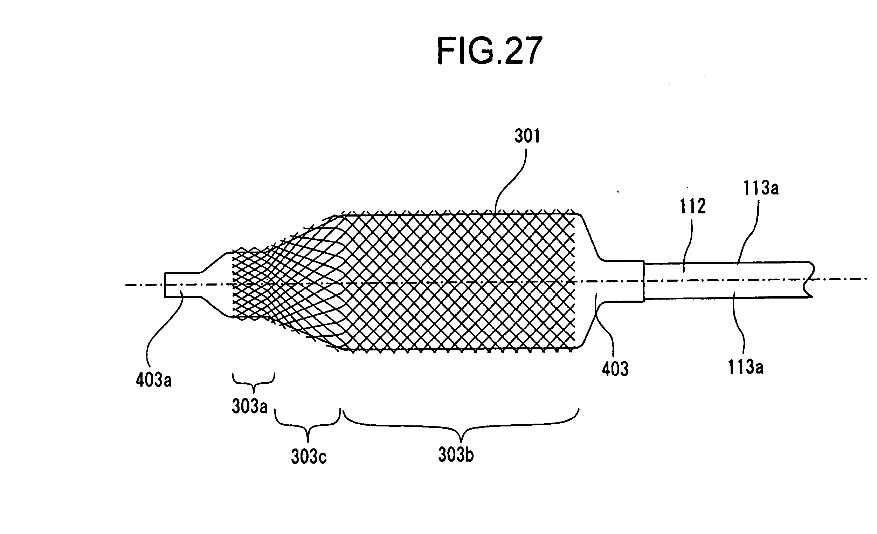

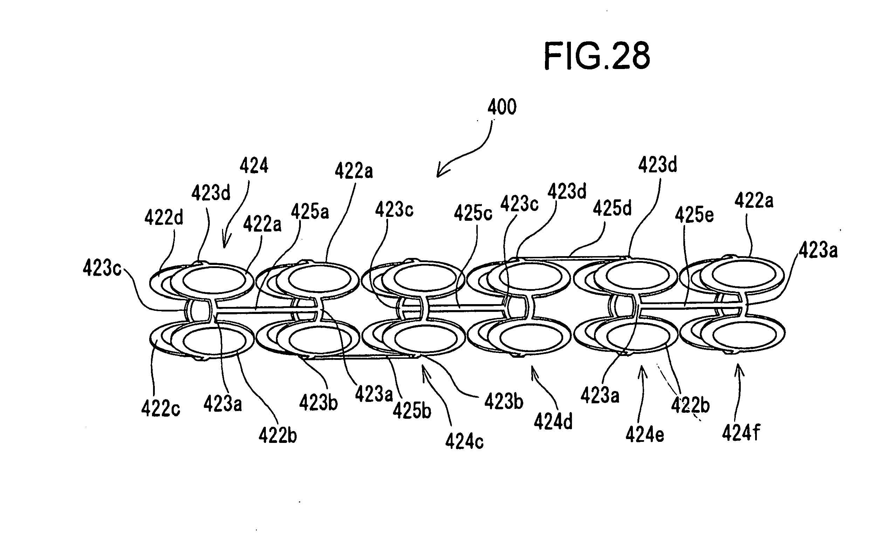

Fig. 22 is a front view of a further possible construction of the embolus member capturing device leaving implement, which does not form a part of the present invention. Fig. 23 is a partly broken enlarged view of a distal end portion of the embolus member capturing device leaving implement shown in Fig. 22. Fig. 24 is a view showing an expanded state of an expansion body of the embolus member capturing device leaving implement in the condition where an embolus member capturing device is not mounted. Fig. 25 is a view showing the condition where the embolus member capturing device is expanded by the expansion body of the embolus member capturing device leaving implement Fig. 26 is a view showing an expanded state of an expansion body in the condition where an embolus member capturing device is not mounted, in an embolus member capturing device leaving implement according to an embodiment of the present invention. Fig. 27 is a view showing the condition where the embolus member capturing device is expanded by the expansion body of the embolus member capturing device leaving implement according to the embodiment shown in Fig. 26. Figs. 28 to 31 are front views of examples of the embolus member capturing device used for the embolus member capturing device leaving implement.

The embolus member capturing device leaving implement 300 according to this possible construction includes a catheter 302 and an embolus member capturing device 301. The catheter 302 has an expandable and foldable expansion body 303 provided in the vicinity of a distal end portion thereof. The embolus member capturing device 301 is composed of a tubular body mounted on the expansion body 303 of the catheter 302 and having one end portion and the other end portion. At least the proximal side 301b relative to a central portion of the embolus member capturing device 301 is mounted to the outside surface of an expandable portion of the expansion body 303, and the one end portion 301a of the embolus member capturing device 301 envelopes the outside surface of a distal side non-expandable portion of the expansion body 303 or the outside surface of the catheter 302 on the distal side relative to the distal end of the expansion body 303.

The outside surface of the distal side non-expandable portion and a tapered expandable portion (taper-shape expandable portion) 303e of the expansion body 303, which is enveloped by the one end portion 301a of the embolus member capturing device 301, or the outside surface of the catheter 302 on the distal side relative to the distal end of the expansion body 303 is preferably a low-friction surface. The low-friction surface can be formed, for example, by applying a lubricating material (e.g., silicone oil) to the surface, or by coating the surface with a low-friction material (e.g., fluoro-resin).

The embolus member capturing device leaving implement 300 includes the catheter 302 including the expansion body 303, and the embolus member capturing device 101 so mounted as to envelope the expansion body 303.

As shown in Fig. 22 and in Fig. 12 referred to in view of the same configuration, in the embolus member capturing device leaving implement 300 according to this possible construction, the catheter 302 includes a guide wire lumen 115. One end of the lumen 15 is opened at the distal end of the catheter 302, and the other end is opened at a proximal end portion of the catheter 302.

The catheter 302 includes an inner tube 112, an outer tube 113, and a branched hub 110. As shown in Figs. 23,24, and 12, the inner tube 112 is a tube body provided therein with a guide wire lumen 115 for passing a guide wire therethrough. The inner tube 112 and the outer tube 113 are the same as in the above-described possible constructions.

Also in the embolus member capturing device leaving implement 300 in this possible construction, like in the embolus member capturing device leaving implement 100 in the above-described possible construction, the outer tube 113 is composed of a distal side outer tube 113a and a main body side outer tube 113b. Both of the tubes 113a and 113b are joined to each other. The distal side outer tube 113a is reduced in diameter in a taper form at its portion on the distal side relative to its joint portion for joining to the main body side outer tube 113b, and has a small diameter on the distal side relative to the tapered portion.

The expansion body 303 has a distal side joint portion 303a and a proximal side joint portion 303b. The distal side joint portion 303a is fixed to a distal end portion (specifically, a position slightly on the proximal side relative to the distal end) of the inner tube 112. The proximal side joint portion 303b is fixed to a distal end portion of the outer tube 113. In addition, the expansion body 303 is communicated with an expansion body expanding lumen 116 in the vicinity of a proximal end portion thereof. The material for forming the inner tube 112 and the outer tube 113 is the same as described above.

As shown in Fig. 23, the expansion body 303 is foldable. When the expansion body 303 is not expanded, the expansion body 303 can be folded onto the outer circumference of the inner tube 112. As shown in Fig. 24, the expansion body 303 has an expandable portion 303d forming a tubular portion (preferably, a hollow cylindrical portion) having a substantially equal diameter so as to be capable of expanding the embolus member capturing device 301 to be mounted thereto. The hollow cylindrical portion may not necessarily be a perfect hollow cylinder, and may have a polygonal columnar shape. Further, the expansion body 303 includes a tapered expandable portion (taper-shape expandable portion) 303e located on the distal side relative to the expandable portion 303d. Furthermore, of the expansion body 303, the distal side joint portion 303a is attached liquid-tight to the inner tube 112, whereas the proximal side joint portion 303b is attached liquid-tight to the distal end of the outer tube 113, by an adhesive, by fusing, or the like. In addition, a distal end portion of the expansion body 303 is in close contact with a contrast member 117, and this close contact portion is a non-expandable portion. The expansion body 303 forms an expansion space 303c between the inside surface thereof and the outside surface of the inner tube 112. The expansion space 303c is communicated, at its proximal end portion, with the expanding lumen 116 over the entire circumference thereof. The material for forming the expansion body 303 and the size of the expansion body 303 are the same as described above.

The catheter 302 includes two contrast members 117 and 118 fixed to the outside surface of the catheter 302 at respective positions corresponding to both end portions of the embolus member capturing device 101 to be mounted thereto. Each of the contrast members 117, 118 is preferably a ring-shaped one, one formed by winding a filamentous material in a coil shape, or the like having a predetermined length. Examples of the preferable material for forming the contrast members 117, 118 include gold, platinum, tungsten, alloys thereof, and silver-palladium alloys.

The branched hub 110 is fixed to the proximal end of the embolus member capturing device leaving implement 300 in this possible construction, as shown in Fig. 12. The structure of a proximal end portion of the embolus member capturing device leaving implement 300 inclusive of the branched hub 110 is the same as in the embolus member capturing device leaving implement 100 described above. Therefore, the above description should be referred to.

Incidentally, the structure of the proximal end of the embolus member capturing device leaving implement 300 is not limited to the above-described. For example, a structure may be adopted in which the branched hub 110 is not provided, and tubes having a port member for forming an opening portion at the proximal end thereof are attached liquid-tight to the guide wire lumen 115 and the expansion body expanding lumen 116, respectively.

Besides, the embolus member capturing device leaving implement is not limited to the over-the-wire type one such as the embolus member capturing device leaving implement 300, and may be one of the type in which a catheter is provided at its intermediate portion with a guide wire insertion port (so-called rapid exchange type) as shown in Figs. 13 to 21 and described above. The above-described embolus member capturing device leaving implement 200 includes the sheath 205 through which the catheter 210 with the embolus member capturing device mounted thereto can be passed. In the embolus member capturing device leaving implement 300, also, a sheath through which the catheter can be passed may be provided. Besides, the embolus member capturing device leaving implement 300 in this possible construction may be one applied to the type in which a catheter is provided at its intermediate portion with a guide wire insertion port (so-called rapid exchange type) shown in Figs. 13 to 21 and described above, wherein the sheath 205 is not provided.

An embolus member capturing device 301 is mounted onto the expansion body 303 of the catheter 302. At least the other end side 301b relative to a central portion of the embolus member capturing device 301 is located on the outside surface of the expandable portion of the expansion body 302. Further, one end portion 301a of the embolus member capturing device 301 is located on the outside surface of the distal side non-expandable portion of the expansion body 303 or the outside surface of the catheter 302 on the distal side relative to the distal end of the expansion body 303. Therefore, when the expansion body 303 is expanded, as shown in Fig. 25, the embolus member capturing device 301 is expanded to the state of having the one end portion 301a substantially unchanged in outside diameter, the other end portion 301b radially enlarged into a hollow cylindrical shape, and an intermediate portion 301c radially enlarged into a tapered shape between the one end portion 301a and the other end portion 301b. The embolus member capturing device 301 left to indwell in a blood vessel captures an embolus member (e.g., embolus coil) at its one end portion 301a substantially unchanged in outside diameter or at its intermediate portion 301c.

One end portion 301a of the embolus member capturing device 301 may be located on the outside surface of the tapered expandable portion 303e. Therefore, when the expansion body 303 is expanded, the embolus member capturing device 301 may be expanded to the state of having the one end portion 301a radially enlarged into a tapered shape in outside diameter, the other end portion 301b radially enlarged into a hollow cylindrical shape. The embolus member capturing device 301 left to indwell in a blood vessel captures an embolus member (e.g., embolus coil) at its one end portion 301a radially enlarged into a tapered shape in outside diameter.

In addition, as shown in Fig. 23, the embolus member capturing device 301 is preferably mounted to the catheter 302 in such a manner that its one end is located at the distal end of the contrast member 117 and its other end is located on the proximal end of the contrast member 118. Besides, it is preferable that the outside diameter of the other end portion of the embolus member capturing device 301 upon expansion can be regulated by the expansive force of the expansion body 303.

As shown in Figs. 24 and 25, it is preferable that the proximal end (the right end in Fig. 25) of the other end portion 301b of the embolus member capturing device 301 is located at least on the proximal side relative to a central portion of the hollow cylindrical portion (in Fig. 24, the expandable portion 303d) of the expansion body 303. This configuration prevents the embolus member capturing device 301 from slipping off during when the expansion body 303 is expanded. Specifically, when an expanding fluid (e.g., a contrast agent) is injected into the inside of the expansion body 303, the expansion body 303 is gradually expanded from the proximal side toward the distal side. A force tending to push out the embolus member capturing device 301 toward the distal side is exerted on the embolus member capturing device 301, and the embolus member capturing device 301 may slip off from the expansion body 303. However, since the proximal end of the other end portion 301b is located on the proximal side relative to the central portion of the hollow cylindrical portion of the expansion body 303, the area of contact between the hollow cylindrical portion of the expansion body 303 and the other end portion 301b is large. The embolus member capturing device 301 can be prevented from slipping off at the time of expansion. In the example shown in Fig. 25, the proximal end of the other end portion 301b of the embolus member capturing device 301 is located nearly at the proximal end of the hollow cylindrical portion of the expansion body 303.

The embolus member capturing device 301 may be any one, inasmuch as it can be expanded by expansion of the expansion body 301 and it can be fixed to a blood vessel when expanded. For example, the embolus member capturing device 301 may be a knitted form one as shown in Fig. 25, and may be any of those shown respectively in Figs. 28 to 31.

The embolus member capturing device 301 shown in Fig. 25 is a hollow cylindrical net member knitted from metallic thin wires. The expansion body 301 is spread out, and the mesh openings are spread or deformed, whereby the embolus member capturing device 301 is changed in shape.

An embolus member capturing device 400 shown in Fig. 28 is composed of a tubular body having one end portion and the other end portion and having a substantially constant first outside diameter. The capturing device 400 is expandable when a radially dilating force is exerted from the inside of the tubular body.

The embolus member capturing device 400 is expandable in correspondence with the shape of the expansion body upon expansion. Therefore, the one end portion of the embolus member capturing device 400 is substantially not expanded by the expansion body. The capturing device 400 substantially maintains the outside diameter thereof before expansion, whereas the other end portion is expanded by the expansion body. Of the embolus member capturing device 400, either the one end portion or an intermediate portion between the one end portion and the other end portion forms an embolus member capturing portion. Incidentally, it is preferable for the intermediate portion to form an embolus member capturing portion which has an inside diameter varying from the one end side toward the other end side upon expansion of the expansion body.

As shown in Fig. 28, the embolus member capturing device 400 is composed of annular units 424 (424a, 424b, 424c, 424d). Each of the units 424 has elliptic or polygonal (in the figure, elliptic) component elements 422 (422a, 422b, 422c, 422d). The elements 422 elongate in the axial direction of the embolus member capturing device 400, are opened at a central portion thereof, and are arranged on a circular circumference at nearly equal angular intervals around the center axis of the embolus member capturing device 400. The adjacent portions (side portions) in the circumferential direction of the component elements are connected to each other through connection portions 423 (423a, 423b, 423c, 423d). A plurality of the annular units 424a, 424b, 424c, 424d are arrayed in the axial direction of the embolus member capturing device 400. Further, the connection portion(s) 423 of each annular unit 424 and the connection portion(s) 423 of the adjacent annular unit 424 are preferably linked to each other through link portion(s) 425 (425a, 425b, 425c) with at least one location. Incidentally, the configuration of the embolus member capturing device 400 is the same as that of the other end portion and the intermediate portion of the embolus member capturing device 10 shown in Figs. 1 and 2 and described above. Therefore, for the detailed configuration of the embolus member capturing device 400, the description concerning the embolus member capturing device 10 should be referred to.

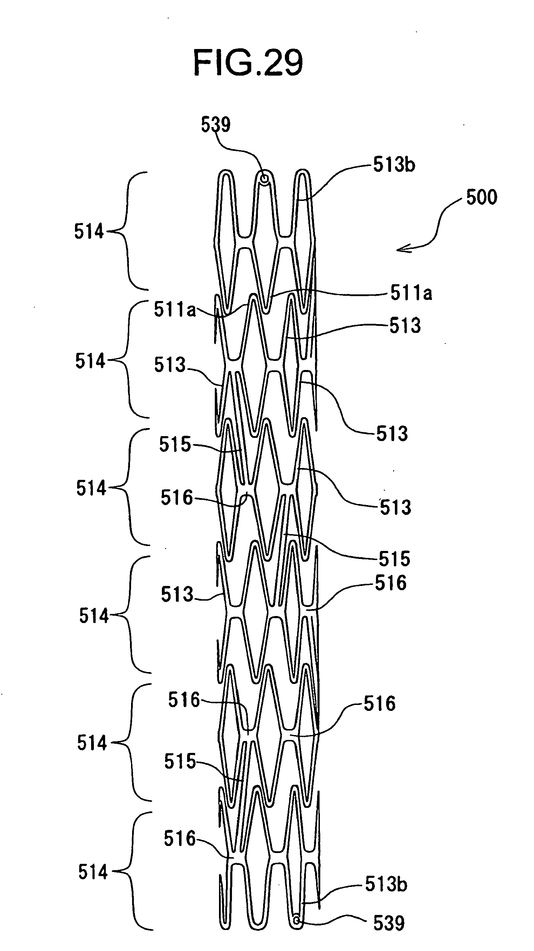

The embolus member capturing device may be in the form as shown in Fig. 29.

The embolus member capturing device 500 is formed to be a tubular body, has a diameter suitable for insertion into a blood vessel, and is expandable when a radially dilating force is exerted from the inside of the tubular body. The embolus member capturing device 500 is composed of annular units 534. A plurality of polygonal filamentous bodies 533 each have a multiplicity of filamentous bent portions and an opening so as to be extended when a radially dilating force is exerted thereon. The filamentous bodies 533 are connected to each other through a plurality of connection portions 536. A plurality of the annular units 534 are arrayed in the axial direction of the embolus member capturing device 500. The embolus member capturing device 500 includes link portions 535 linking the adjacent annular units 534 to each other via the connection portions 536 and being not continuous in the axial direction of the embolus member capturing device 500. The link portions 535 are provided between the adjacent annular units 534 in plurality and at opposite positions or at substantially equal angular intervals around the center axis of the embolus member capturing device 500. Each of the polygonal filamentous bodies 533 is extended when a radially dilating force is exerted thereon, resulting in an increase in the outside diameter.

In this embolus member capturing device 500, each of the polygonal filamentous bodies 533 is in a collapsed shape elongate in the axial direction of the embolus member capturing device 500. Further, as in this embolus member capturing device 500, it is preferable that an outside portion of each of the polygonal filamentous bodies 533 located at both ends of the embolus member capturing device 500 is semi-elliptic in shape. Furthermore, as in the embolus member capturing device 500 according to this possible construction, it is preferable that vertex portions of the bent portions 531a of the polygonal filamentous bodies 533 in each annular unit 534 are located in the spaces formed between the bent portions 531a of the polygonal filamentous bodies 533 in the adjacent annular unit 534.

Incidentally, the embolus member capturing device 500 is the same as the embolus member capturing device 30 shown in Figs. 3 and 4 and described above, except that the embolus member capturing device 500 does not include extension preventive portions. Therefore, for the detailed configuration of the embolus member capturing device 500, the description concerning the embolus member capturing device 30 should be referred to.

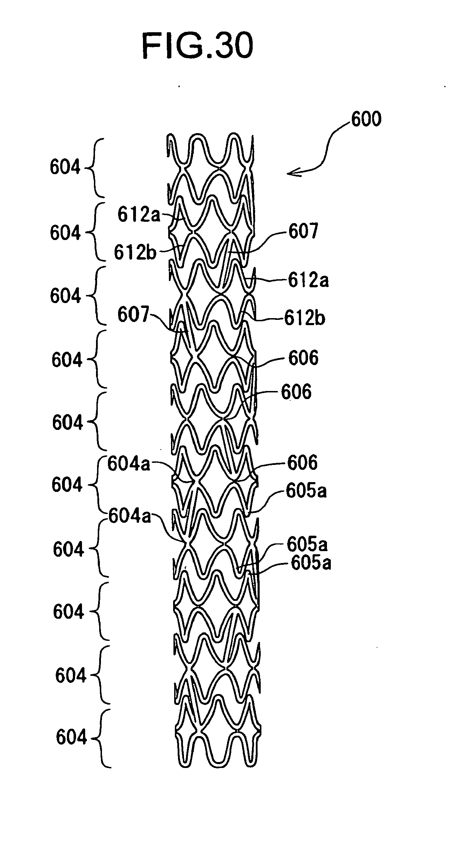

The embolus member capturing device may be in the form as shown in Fig. 30.

This embolus member capturing device 600 is an embolus member capturing device which is formed to be a tubular body, has a diameter suitable for insertion into a lumen in a living body, and is expandable when a radially dilating force is exerted from the inside of the tubular body. The embolus member capturing device 600 is composed of annular units 644 each including a first wavy annular body 642a, a second wavy annular body 642, and a plurality of connection portions 646. The first wavy annular body 642a is formed in an annular shape from a wavy element having a multiplicity of bent portions 645a. The second wavy annular body 642b is disposed in the axial direction of the embolus member capturing device 600 to have its mount portions close to valley portions of the first wavy annular body 642 and which is formed in an annular shape from a wavy element having filamentous bent portions 645b. The connection portions 646 connects the valley portions of the first wavy annular body 642a and the mount portions of the second wavy annular body 642b to each other. A plurality of the annular units 644 are arrayed in the axial direction of the embolus member capturing device 600, and the embolus member capturing device 600 includes link portions 647 for linking the adjacent annular units 644 to each other via connection portion forming sites. Further, the link portions 647 are provided between the adjacent annular units 644 in plurality and at opposite positions or at substantially equal angular intervals around the center axis of the embolus member capturing device 600.

Incidentally, the embolus member capturing device 600 is the same as the embolus member capturing device 40 shown in Figs. 5 and 6 and described above, except that the embolus member capturing device 600 does not include extension restrictive portions.

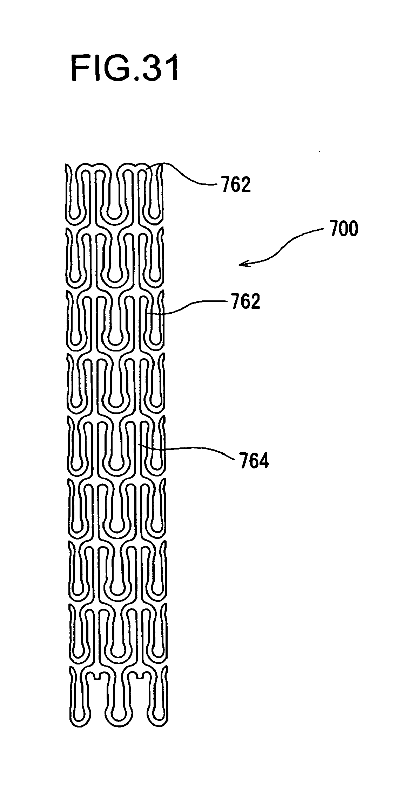

The embolus member capturing device may be in the form as shown in Fig. 31.

In this embolus member capturing device 700, as shown in Fig. 31, a plurality of wavy annular bodies 762 are so arranged that mount portions or valley portions of the wavy annular bodies 762 adjacent to each other in the axial direction are arrayed substantially rectilinearly in the axial direction. In addition, connection portions 764 are provided for connection between the mount portions or the valley portions of the adjacent wavy annular bodies 762.

In other words, the embolus member capturing device 700 is composed of a plurality of the annular bodies 762 each composed of a wavy (zigzag) annularly continuous (endless) filamentous body for playing the role of maintaining expansion. These annular bodies 762 are connected to each other by the connection portions (connectors) 764 so that the adjacent annular bodies 762 will not part from each other.

The embolus member capturing device leaving implement according to the present invention has a structure in which, as shown in Figs. 26 and 27, an expansion body 403 includes a proximal side expandable portion 403d expandable to a hollow cylindrical shape having a first outside diameter, a distal side expandable portion 403f expandable to a second outside diameter smaller than the first outside diameter, and an intermediate expandable portion 403e expandable to a shape having a diameter reduced toward the distal side between the proximal side expandable portion 403d and the distal side expandable portion 403f. An embolus member capturing device 301 is mounted to the expansion body 403 so that the other end portion 301b of the capturing device 301 envelopes the proximal side expandable portion 403d, one end portion 301a of the capturing device 301 envelopes the distal side expandable portion 403f, and the portion between the one end portion 301a and the other end portion 301b envelopes the intermediate expandable portion 403e.

The embolus member capturing device leaving implement in this embodiment is substantially the same in configuration as the above-described embolus member capturing device leaving implement 300, except for the shape of the expansion body; therefore, the above description should be referred to, and only the differences from the embolus member capturing device leaving implement 300 will be described.

In the embolus member capturing device leaving implement according to this embodiment, as the expansion body, an expansion body 403 shown in Fig. 26 is used. The expansion body 403 is foldable, and can be folded onto the outer circumference of an inner tube 112 when not expanded. As shown in Fig. 26, the expansion body 403 has a proximal side expandable portion 403d. The expandable portion 403d is a tubular portion (preferably, a hollow cylindrical portion) having a substantially constant diameter (a first outside diameter) so as to be capable of expanding an embolus member capturing device 301 to be mounted thereto. The hollow cylindrical portion may not necessarily be a perfect hollow cylinder, and may have a polygonal columnar shape. The expansion body 403 includes a tapered expandable portion 403e located on the distal side relative to the expandable portion 403d, and a distal side expandable portion (small diameter expandable portion) 403f which is located on the distal side relative to the tapered expandable portion 403e. The expandable portion 403f is expandable to a second outside diameter smaller than the first outside diameter.

The embolus member capturing device 301 is mounted onto the expansion body 403 of a catheter 302. At least the other end side 303b relative to a central portion of the embolus member capturing device 301 is located on the outside diameter of the expandable portion 403d of the expansion body 403. Further, the one end portion 301a of the embolus member capturing device 301 is located on the distal side small diameter expandable portion 403f of the expansion body 403. Therefore, when the expansion body 403 is expanded, as shown in Fig. 27, the embolus member capturing device 301 is expanded into the state of having the one end portion 301a slightly enlarged in outside diameter, the other end portion 303b enlarged in diameter into a hollow cylindrical shape, and an intermediate portion 303c enlarged in diameter into a tapered shape between the one end portion 303a and the other end portion 303b. The embolus member capturing device 301, when left to indwell in a blood vessel, captures an embolus member (e.g., an embolus coil) at the one end portion 303a or the intermediate portion 303c where the outside diameter is substantially unchanged. With the expansion body 403 thus provided with the small diameter expandable portion 403f, it is facilitated to withdrawn the catheter after expansion of the embolus member capturing device 301.

Furthermore, the embolus member capturing device 301 may have a structure in which the above-mentioned other end portion is expanded to the second outside diameter greater than the first outside diameter when a radially dilating force is exerted from the inside of the tubular body. The one end portion is slightly expanded from the first outside diameter or substantially maintains the first outside diameter when a radially dilating force is exerted from the inside of the tubular body. Either the one end portion or the intermediate portion between the one end portion and the other end portion forms an embolus member capturing portion. Specifically, any of the embolus member capturing devices 10, 30, 40, 60 may be used.

As shown in Figs. 26 and 27, the embolus member capturing device 301 has the proximal end (the right end in Fig. 27) of the other end portion 303b located at least on the proximal side relative to a central portion of the hollow cylindrical portion (in Fig. 26, the expandable portion 403d) of the expansion body 403. This configuration prevents the embolus member capturing device 301 from slipping off during when the expansion body 403 is expanded. Specifically, since the proximal end of the other end portion 303b is located on the proximal side relative to the central portion of the hollow cylindrical portion of the expansion body 403, the area of contact between the hollow cylindrical portion of the expansion body 403 and the other end portion 303b is large. Thus, the slipping-off at the time of expansion can be prevented from occurring. In the example shown in Fig. 27, the proximal end of the other end portion 303b of the embolus member capturing device 301 is located nearly at the proximal end of the hollow cylindrical portion of the expansion body 403.

The embolus member capturing device used in the embolus member capturing device leaving implement according to the present invention as above-described preferably has a diameter, in a non-expanded state, of 3.5 mm or less, more preferably in the rang of 1.5 to 2.8 mm. In addition, the diameter of the embolus member capturing device upon expansion is preferably in the range of 2.0 to 6.0 mm. Besides, the whole length of the embolus member capturing device is preferably 30 mm or less, more preferably in the range of 8 to 15 mm.

In addition, the embolus member capturing device 301 preferably has a structure in which the material thickness of the one end portion 303a is smaller than the material thickness of the other end portion 303b. At the time of insertion into a blood vessel of the embolus member capturing device leaving implement with the embolus member capturing device 301 mounted to the expansion body, the one end portion 303a displays flexibility to facilitate the insertion. This is because the material thickness of the one end portion 303a is smaller than the material thickness of the other end portion 303b. It is more preferable that the material thicknesses of the one end portion 303a and the intermediate portion 303c are smaller than the material thickness of the other end portion 303b.

The material for forming the embolus member capturing device is preferably a material having a certain degree of bio-compatibility. Examples of the material include stainless steels, tantalum or tantalum alloys, platinum or platinum alloys, gold or gold alloys, and cobalt-based alloys. Alternatively, a blank processed into the shape of the embolus member capturing device may be plated with a noble metal (gold, platinum). Among stainless steels, preferred is SUS316L having the highest corrosion resistance.

Furthermore, it is preferable that the blank processed to the final shape of the embolus member capturing device is subjected to annealing. The annealing enhances the flexibility and plasticity of the embolus member capturing device as a whole, thereby promising good indwelling performance in a bent blood vessel. As compared with the case of not performing annealing, the annealing reduces the restoring force of the expanded embolus member capturing device for restoring to the shape thereof before expansion, particularly, the returning force for returning to a rectilinear shape. The returning force is developed when the embolus member capturing device is expanded in a bent blood vessel site. Therefore, the physical stimulus exerted on the inside wall of a bent blood vessel is reduced, whereby a cause of restenosis can be reduced. For preventing an oxide film from being formed on the surface of the embolus member capturing device, the annealing is preferably carried out by heating to a temperature of 900 to 1200 degree C in an inert gas atmosphere (e.g., argon gas), followed by slow cooling.

In addition, it is preferable that the embolus member capturing device is chamfered. The chamfering of the embolus member capturing device can be conducted by chemical polishing, electropolishing, or mechanical polishing, after processing to the final shape of the embolus member capturing device. The chemical polishing is preferably carried out by immersion in a stainless steel chemical polishing liquid. The stainless steel chemical polishing liquid may be any liquid that can dissolve the relevant stainless steel; for example, a liquid containing a hydrochloric acid-nitric acid mixture as a basic component and to which an organic sulfur compound and a surfactant have been added for control of dissolution rate, smoothing, and imparting a lustrous property is preferably used.

Furthermore, each of the embolus member capturing devices according to all embodiments described above is preferably provided with a contrast marker or markers, as in the case of the embolus member capturing device 500 shown in Fig. 29. The contrast markers or markers are preferably provided at an end portion or end portions of the embolus member capturing device. Particularly, it is preferable that the contrast markers are provided respectively at both end portions of the embolus member capturing device. Specifically, as in the case of the embolus member capturing device 500 shown in Fig. 29, it is preferable to provide a plurality of contrast markers 539 in the axial direction on one end side (specifically, the distal end side), and to provide a contrast marker 539 on the other end side (specifically, on the proximal end side). This configuration facilitates checking of the positions of the end portions of the embolus member capturing device.

In the contrast marker shown in Fig. 29, a small opening formed in the embolus member capturing device is plugged up with the contrast marker and is fixed to the embolus member capturing device. Such a marker is preferably attached, for example, by a method in which a disc form member of a contrast material slightly smaller than the small opening formed in the embolus member capturing device is placed in the small opening, and the disc form member is caulked by pressing it from both sides. Incidentally, the contrast marker is not limited to the above-mentioned, and may be any one. For example, there may be used a contrast marker formed by coating an outside surface of the embolus member capturing device with a contrast material, a contrast marker formed by winding a wire of a contrast material, a contrast marker formed by attaching a ring-shaped member of a contrast material, or the like. Examples of the material for forming the contrast markers include gold, platinum, tungsten, alloys thereof, silver-palladium alloys, etc. Incidentally, the contrast markers include those for radiography, those for sonography, and those for NMR imaging.

Furthermore, the embolus member capturing device preferably has a structure in which a part or the whole part thereof is coated with a highly thrombus-generating material, or a fibrous member formed of a highly thrombus-generating material is attached to a part or the whole part thereof.

Examples of the highly thrombus-generating material include silk yarn, polyesters (e.g., Dacron), nylon, and PET. It is preferable to coat the inside surface of the embolus member capturing device with such a material. Though the inside surface of the embolus member capturing device may be entirely coated, the inside surfaces of the one end portion and the intermediate portion may be coated, or only the inside surface of the intermediate portion may be coated. Alternatively, it is preferable to form the highly thrombus-generating material into a fibrous member and to attach the fibrous member to the inside surface of the embolus member capturing device. Though a plurality of the fibrous members may be attached to the whole part of the inside surface of the embolus member capturing device, the fibrous members may be attached to the inside surfaces of the one end portion and the intermediate portion, or the fibrous member may be attached only to the inside surface of the intermediate portion. Also, the fibrous members may be provided on the outside surfaces of the one end portion and the intermediate portion, or only on the outside surface of the intermediate portion. For the attachment, there may be used a method of adhering the fibrous member to the embolus member capturing device by use of an adhesive or the like, a method of forming a cut in a part of the embolus member capturing device by laser or the like and clamping the fibrous member in the cut, or the like.

In addition, it is preferable that, in the embolus member capturing devices, the second outside diameter on the other end side upon expansion can be regulated by the radially dilating force exerted from the inside of the tubular body. Namely, in these embolus member capturing devices, the diameter upon expansion on the other end side is not particularly limited, on a configuration basis. Therefore, the second outside diameter on the other end side upon expansion can be regulated by the radially dilating force exerted from the inside of the tubular body, i.e., by the expansive force of the expansion body to which the embolus member capturing device is mounted.

In the next place, one possible construction of a blood vessel embolus member the present invention will be described referring to the drawings.

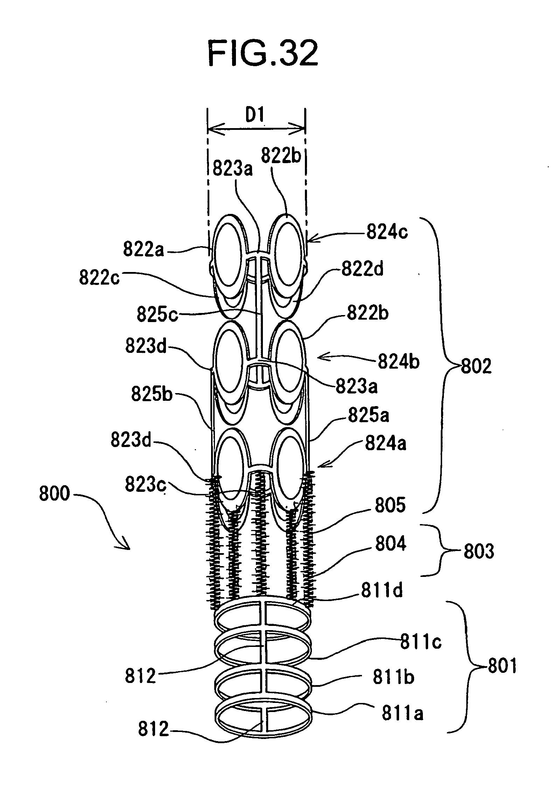

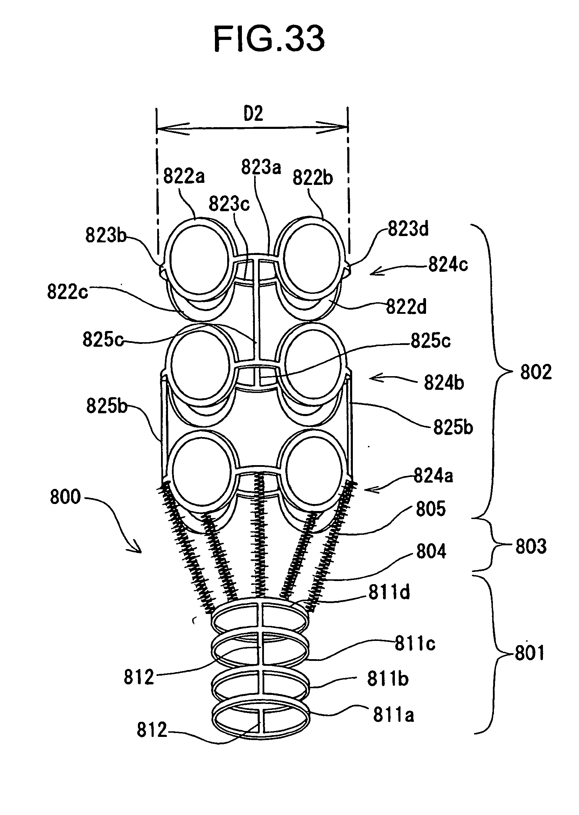

Fig. 32 is a perspective view of the blood vessel embolus member according to a possible construction, which does not form a part of the present invention. Fig. 33 is a view showing the condition where the blood vessel embolus member is expanded by an expansion body of an embolus member capturing device.

The blood vessel embolus member 800 is composed of a tubular body having one end portion 801, the other end portion 802, and an intermediate portion 803 located between the one end portion 801 and the other end portion 802. At least the inside surface of the intermediate portion 803 is a thrombus formation promoting surface.

The blood vessel embolus member 800 in the possible construction shown in Figs. 32 and 33 has a structure in which at least either one of the one end portion and the other end portion, specifically, the other end portion 802 is expandable when a radially dilating force is exerted from the inside of the tubular body (in other words, the other end portion 802) and is capable of making close contact with a blood vessel wall. In addition, the one end portion 801 is slightly expanded from the outside diameter D1 thereof before expansion or substantially maintains the outside diameter D1 thereof before expansion when the radially dilating force is exerted from the inside of the tubular body (the one end portion). The intermediate portion 803 between the one end portion 801 and the other end portion 802 forms an embolus portion.

In the blood vessel embolus member 800 in this possible construction, as shown in Figs. 32 and 33, the intermediate portion 803 is composed of a plurality of coil form members 804 for linking the one end portion 801 and the other end portion 802 to each other, and a thrombus-forming fibrous member 805 is fixed to the surface thereof. The thrombus-forming fibrous member 805 may be fixed by clamping it in the gaps in the coil form members 804, or may be fixed to the surface of the intermediate portion 803 by an adhesive or the like. Besides, the thrombus-forming fibrous member 805 may be fixed only to the side of the inside surface of the intermediate portion 803. As shown in the figures, it is preferable for the thrombus-forming fibrous member 805 to be fixed to the whole surface of the intermediate portion 803. In this blood vessel embolus member 800, each of the coil form members 804 is linking the one end portion 801 and the other end portion 802 to each other. The linking can be carried out, for example, by welding, soldering, or the like. Since the one end portion 801 and the other end portion 802 are thus linked to each other by the coil form members 804, the flexibility possessed by the coil form members 804 facilitates the insertion of the embolus member 800 into a blood vessel. Incidentally, the coil form members 804 preferably are low in spring elasticity, in order to exert no influence on the shape of the other end portion 802 upon expansion.

Incidentally, the intermediate portion 803 is not limited to the above-mentioned coil type; as the intermediate portion 803, there may be used, for example, a net form member composed of a thrombus-forming fibrous material, a member formed by fixing a thrombus-forming fibrous material to the surface of a net form member formed of a metal or a resin, a member formed by fixing a thrombus-forming fibrous material to the surface of a single coil body having a high thermoplastic deformability, or the like. Incidentally, where a net form member composed of a thrombus-forming fibrous material is used, the net form member is preferably produced by a knitting or weaving method that provides the net form member with a comparatively good extension-contraction property.

Examples of the blank material for the thrombus-forming fibrous member or material include silk yarn, polyesters (e.g., Dacron), nylon, and PET. It is preferable to coat the inside surface of the intermediate portion of the embolus member with such a blank material. Incidentally, the whole part of the inside surface of the embolus member or, further, the entire surfaces of the embolus member may be coated with the thrombus-forming fibrous material.

In the blood vessel embolus member 800 in this possible construction, the other end portion 802 is expandable when a radially dilating force is exerted from the inside of the tubular body. Specifically, the other end portion 802 is expanded as shown in Fig. 33 when a radially dilating force is exerted from the inside of the other end portion 802.

In addition, in the embolus member 800 according to this possible construction, the one end portion 801 is non-expandable (non-extensible) when a radially dilating force is exerted from the inside of the tubular body, i.e., when the expansion body is expanded. To be more specific, the one end portion 801 includes a plurality of annular bodies 811 (811a, 811b, 811c, 811d) which are substantially orthogonal to the center axis of the embolus member 800. The plurality of annular bodies 811 are arrayed substantially in parallel to the axial direction of the embolus member capturing device. Further, the plurality of annular bodies 811 are linked to each other by link portions 812 (812a, 812b). The link portions may be provided in plurality as in this possible construction, or only one link portion may be provided. Incidentally, the link portion is not limited to the one which links all the annular bodies 811 rectilinearly as in this embodiment; a link portion, which links the adjacent annular bodies 811 at different positions, may also be used. Also, the one end portion 801 is not limited to the above-mentioned configuration, and may be in the form of a short pipe, for example. Besides, the one end portion may be of the type having extension restrictive portions, as in the cases of the embolus member capturing devices 30, 40, and 60 shown in Figs. 3 to 8 and described above.

In the embolus member 800 according to this possible construction, the other end portion 802 is expandable (extensible) when a radially dilating force is exerted from the inside of the tubular body, i.e., when the expansion body is expanded. As shown in Figs. 32 and 33, the other end portion 802 is composed of annular units 824 (824a, 824b, 824c). Each of the units 824 has elliptic or polygonal (elliptic, in the figures) component elements 822 (822a, 822b, 822c, 822d). The elements 822 elongate in the axial direction of the embolus member 800, are closed at center portions thereof, and are arranged on a circular circumference at substantially equal angular intervals around the center axis of the embolus member 800. The adjacent portions (side portions) in the circumferential direction of the component elements 822 are connected to each other through connection portions 823 (823a, 823b, 823c, 823d). A plurality of the annular units 824a, 824b, and 824c are arrayed in the axial direction of the embolus member 800. Further, the connection portion(s) 823 of each annular unit 824 and the connection portion(s) 823 of the adjacent annular unit 824 are preferably linked to each other through a link portion or link portions 825 (825a, 825b, and 825c) with at least one location.

The length of one annular unit in the embolus member 800, that is, the length in the axial direction of one polygonal filamentous body is preferably in the range of about 1.5 to 4.0 mm, more preferably 2.0 to 3.0 mm. The number of the annular units in the embolus member 800 is preferably in the range of three to ten, more preferably three to eight The material thickness of the annular units in the central portion of the embolus member 800 is preferably in the range of about 0.01 to 0.12 mm, more preferably 0.03 to 0.10 mm. The material thickness of the embolus member 800 is preferably in the range of about 0.01 to 0.12 mm. In the embolus member 800, it is preferable that the material thickness of the one end portion 801 is smaller than the material thickness of the other end portion 802. At the time of insertion into a blood vessel of the embolus member mounted to the expansion body (see Fig. 9), the one end portion 801 displays flexibility to facilitate the insertion, since the material thickness of the one end portion 801 is smaller than the material thickness of the other end portion 802. In addition, the diameter of the embolus member 800 upon molding (before compression) is preferably in the range of about 1.5 to 3.5 mm, more preferably 2.0 to 3.0 mm.

Incidentally, the embolus member 800 in this possible construction as above-described is of the type in which the adjacent annular units are linked to each other through two link portions. Therefore, the link portions are arranged at positions opposite to each other, with the center axis of the embolus member 800 therebetween. However, this configuration is not limitative. Three or four (or, two to four, if the case of two link portions is included) link portions may be provided between the adjacent annular units. In this case, the link portions are arranged at substantially equal angular intervals around the center axis of the embolus member 800. Namely, where three link portions are provided between the adjacent annular units, the link portions are arranged at an angular interval of about 120 degrees.

Incidentally, the other end portion 802 is not limited to the above-described configuration; for example, the other end portion may be of any of the types in the embolus member capturing devices 30, 40, and 60 shown in Figs. 3 to 8 and described above.

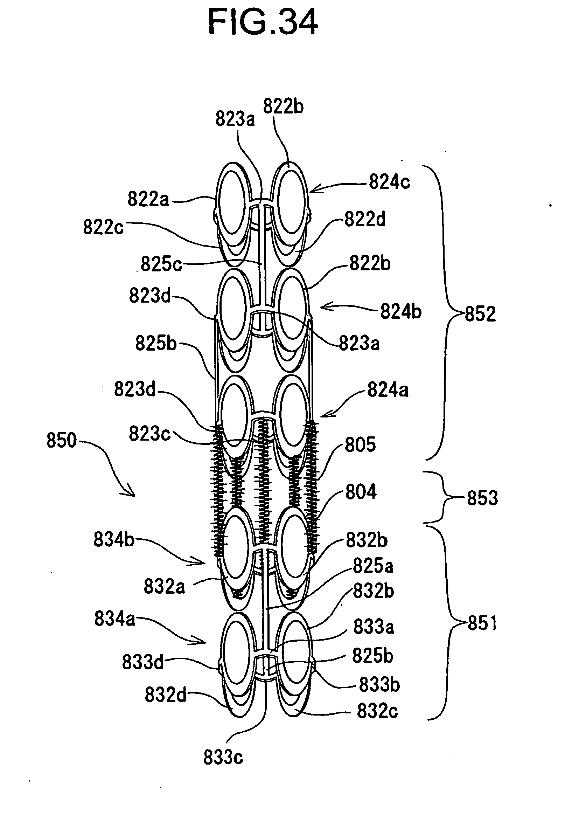

Now, a blood vessel embolus member according to a possible construction shown in Fig. 34 will be described below.

Fig. 34 is a perspective view of the blood vessel embolus member according to another possible construction of the present invention.

The blood vessel embolus member 850 in this possible construction differs from the above-described blood vessel embolus member 800 only in configuration of one end portion 851. In this blood vessel embolus member 850, the one end portion 851 is in the form of being expandable when an expansive force is exerted from the inside thereof.

Therefore, in the same manner as in the embolus member capturing device leaving implement 300 shown in Figs. 22 to 31 and described above, the blood vessel embolus member 850 in this embodiment is mounted on an expandable and foldable expansion body 303 provided in the vicinity of a distal end portion of a catheter 302. At least the other end side 852 relative to a central portion of the embolus member 850 is mounted onto the outside surface of an expandable portion of the expansion body 303. The one end portion 851 of the embolus member 850 is so mounted as to envelope the outside surface of a distal side non-expandable portion of the expansion body 303 or the outside surface of the catheter 302 on the distal side relative to the distal end of the expansion body 303.

Therefore, the one end portion 851 of the embolus member 850 maintains a substantially non-expanded state, whereas the other end portion 852 is expanded to such an extent that it can indwell in a blood vessel. In addition, with the embolus member 850 mounted at the same position as that of the expansion body 403 in the above-described embolus member capturing device leaving implement 400 (see Fig. 26), the one end portion 851 is expanded to a diameter corresponding to the small diameter expandable portion 403f of the expansion body 403. The other end portion 852 is expanded to a diameter corresponding to the proximal side expandable portion 403d of the expansion body 403, thereby enabling the indwelling in the blood vessel. This configuration ensures that a bloodstream collides on the inside surface of an intermediate portion 853 composed of a thrombus-forming material 810, whereby thrombus formation is facilitated and an assured embolus-forming effect is obtained.

The one end portion 851 of the embolus member 850 may be so mounted as to envelope the outside surface of a tapered expandable portion 303e of the expansion body 303. In this case, the one end portion 851 of the embolus member 850 may be enlarged radially into a tapered shape in outside diameter, whereas the other end portion 852 is expanded to such an extent that it can indwell in a blood vessel.

Incidentally, for the positional relationship between the proximal end of the other end portion 852 of the embolus member 850 and the tubular portion of the expansion body 303 or the expansion body 403, the above description should be referred to.

As shown in Fig. 34, the embolus member 850 is composed of annular units 834 (834a, 834b). Each of the units 834 has elliptic or polygonal (in the figure, elliptic) component elements 832 (832a, 832b, 832c, 832d). The elements 832 elongate in the axial direction of the embolus member 850, are closed at a central portion thereof, and are arranged on a circular circumference at substantially equal angular intervals around the center axis of the embolus member 850. Adjacent portions (side portions) in the circular circumferential direction of the component elements are connected to each other through connection portions 833 (833a, 833b, 833c, 833d). A plurality of the annular units 834a, 834b is arrayed in the axial direction of the embolus member 850. Further, it is preferable that the connection portion(s) 833a of the annular unit 834a and the connection portion(s) 833a of the adjacent annular unit 834b are linked to each other through a link portion or link portions 825 (825a, 825b) with at least one location. Incidentally, the configuration of the other end portion 852 and the intermediate portion 853 of this embolus member 850 is the same as that of the embolus member 800 shown in Figs. 32 and 33 and described above. Therefore, for detailed configuration of the embolus member 850, the description concerning the embolus member 800 or the embolus member capturing device 10 should be referred to.

Incidentally, the one end portion 851 is not limited to the above-described configuration; for example, the one end portion may be of any of the types in the embolus member capturing devices 500, 600, 700 shown in Figs. 29 to 31 and described above.

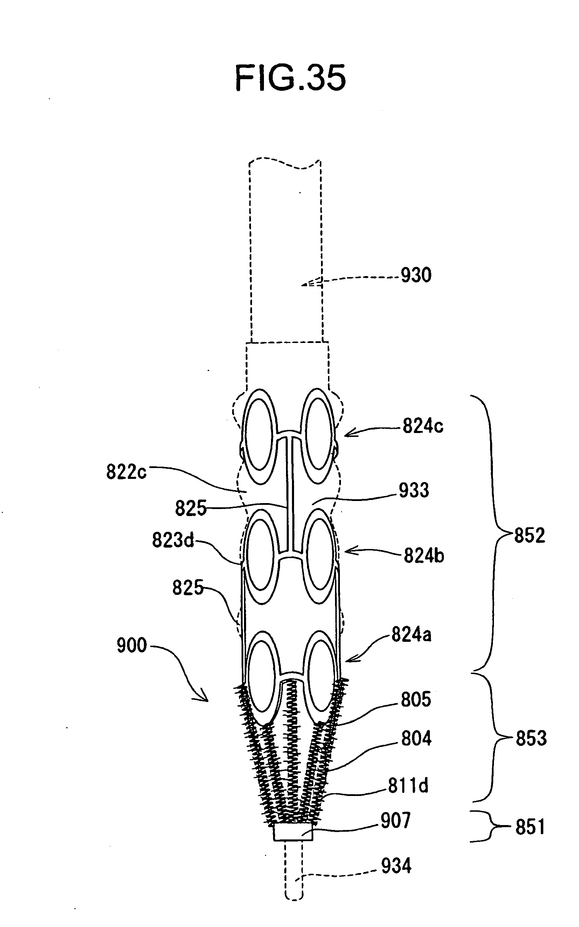

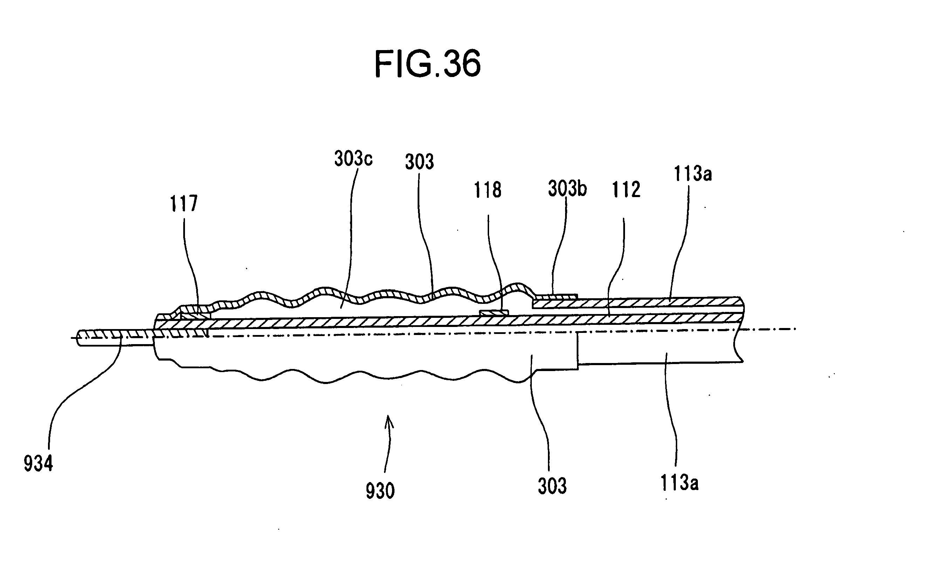

In the next place, a blood vessel embolus member according to a possible construction shown in Figs. 35 and 36 will be described.

Fig. 35 is an illustration of the condition where a blood vessel embolus member according to a further possible construction of the present invention is mounted to an embolus member capturing device leaving implement Fig. 36 is an enlarged sectional view of a distal end portion of an embolus member leaving catheter used in Fig. 35.

The blood vessel embolus member 900 in this possible construction differs from the above-described blood vessel embolus member 800 only in the configuration of one end portion 851. In this blood vessel embolus member 900, the one end portion 851 is composed of a ring-shaped member 907 having a small inside diameter. As shown in Figs. 35 and 36, the ring-shaped member 907 is so disposed as to be located on a guide portion 934 provided at a distal end portion of an embolus member leaving catheter 930. Preferably, the ring-shaped member 907 does not make contact with the guide portion 934 or is slidable relative to the guide portion 934. This configuration facilitates the withdrawing of the catheter 930 after the expansion body 303 is expanded and the other end portion of the embolus member 900 is expanded. Further, in this embolus member 900, the ring-shaped member 907 constituting the one end portion 851 can be formed to have a small diameter, which ensures that a blood vessel can be occluded easily and securely. Examples of the material for forming the ring-shaped member 907 include stainless steels, tantalum or tantalum alloys, platinum or platinum alloys, gold or gold alloys, cobalt-based alloys, and synthetic resins. Alternatively, a blank processed into the shape of the embolus member capturing device may be plated with a noble metal (gold, platinum). Among the stainless steels, preferred is SUS316L having the highest corrosion resistance.

The intermediate portion 853 and the other end portion 852 are the same as in the above-described embolus member 800. Therefore, the above description should be referred to.

The embolus member leaving catheter 930 is the same as the above-described catheter 302, except that the guide portion 934 is provided at the distal end portion of the embolus member leaving catheter 930. In addition, also in the catheter 302, a branched hub 110 is fixed to the proximal end, like in the case of the one shown in Fig. 12. The configuration of a proximal end portion of the catheter 302 including the branched hub is the same as that in the embolus member capturing device leaving implement 100. Therefore, the above description should be referred to.

Incidentally, the structure of the proximal end of the embolus member leaving catheter 302 is not limited to the above-described one; for example, there may be adopted a structure in which the branched hub 110 is not provided, and tubes having a port member for forming an opening portion at the proximal end thereof are attached liquid-tight to a guide wire lumen 115 and an expansion body expanding lumen 116, respectively.

Besides, as the guide portion 934, there is used a coil spring, an elastic metal wire (e.g., super-elastic metal wire), or the like.

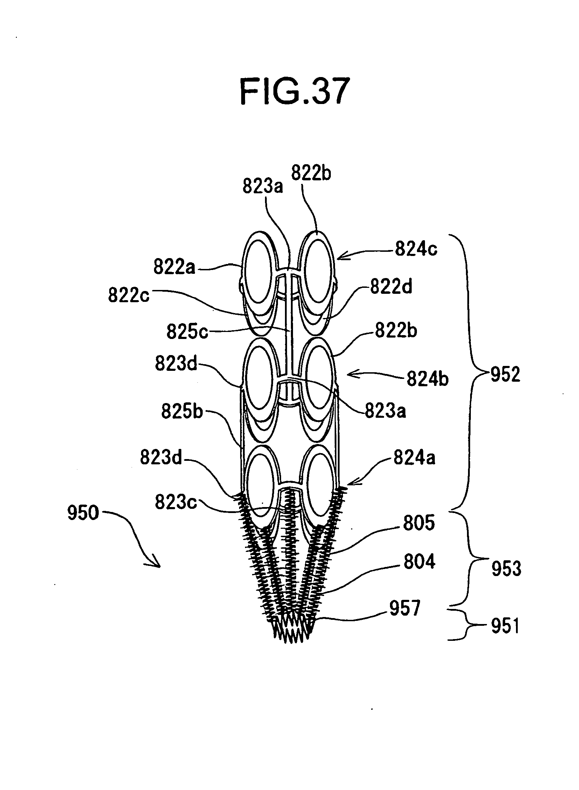

Now, a blood vessel embolus member according to a possible construction shown in Fig. 37 will be described below.

The blood vessel embolus member 950 in this possible construction differs from the above-described blood vessel embolus member 800 only in the configuration of one end portion 951. In this blood vessel embolus member 950, the one end portion 951 is composed of an elastic ring-shaped member 957 having a small diameter. As the elastic ring-shaped member 957, there can be used an endless annular body formed in a zigzag shape from a thin wire, an annular body formed of an elastic material (e.g., rubber, elastomer), or the like, as shown in Fig. 37. The embolus member 950 is disposed at a distal end portion of the embolus member leaving catheter 930 described above and shown in Figs. 35 and 36, in such a manner that the elastic ring-shaped member 957 is located on the guide portion 934 and the other end portion is located on the expandable portion of the expansion body. As the catheter, there may be used the one shown in Figs. 9 to 13, the one shown in Figs. 14 to 21, the one shown in Figs. 22 and 23 or Fig. 26, or the like. In the embolus member 950 in this possible construction, even if the elastic ring-shaped member 957 is disposed on the expandable portion of the expansion body, when the expansion of the expansion body is finished and the expansion body is contracted, the original diameter is restored, so that the catheter can be withdrawn.

In this case, the blood vessel embolus member 950 is mounted on the expansion body of the catheter, the catheter is inserted into a blood vessel, the embolus member 950 is allowed to indwell in a target site, and the catheter is withdrawn. In such a case, the one end portion 951 is contracted and the bloodstream flow region becomes extremely narrow, so that thrombus formation is facilitated and an assured embolus-forming effect is obtained.

Examples for forming the embolus members in all the possible constructions described above include stainless steels, tantalum or tantalum alloys, platinum or platinum alloys, gold or gold alloys, and cobalt-based alloys. Alternatively, a blank material processed into the shape of the embolus member may be coated with a noble metal (gold, platinum). Among the stainless steels, preferred is SUS316L having the highest corrosion resistance.

Further, the blank material processed into the final shape of the embolus member is preferably subjected to annealing. The annealing enhances the flexibility and plasticity of the embolus member as a whole, thereby promising a good indwelling performance in a bent blood vessel. As compared with the case of not conducting annealing, the annealing reduces the restoring force of the expanded embolus member for restoring its shape before expansion, particularly the returning force for returning into a rectilinear shape, which is developed when the embolus member is expanded in a bent blood vessel site. The physical stimulus exerted on the inside wall of the bent blood vessel is reduced, and a cause of restenosis can be reduced. The formation of an oxide film on the surface of the embolus member is prevented. The annealing is preferably carried out by heating to a temperature of 900 to 1200 degree C in an inert gas atmosphere (e.g., argon gas), followed by slow cooling.

In addition, the embolus member is preferably chamfered. The chamfering of the embolus member can be conducted by chemical polishing, electropolishing, or mechanical polishing, after processing into the final shape of the embolus member. The chemical polishing is preferably carried out by immersion in a stainless steel chemical polishing liquid. The stainless steel chemical polishing liquid may be any liquid that can dissolve the relevant stainless steel; for example, a liquid containing a hydrochloric acid-nitric acid mixture as a basic component and to which an organic sulfur compound and a surfactant have been added for control of dissolution rate, smoothing, and imparting a lustrous property is preferably used.

Further, each of the embolus members in all the possible constructions described above is preferably provided with a contrast marker or markers, as in the case of the embolus member capturing device 30 shown in Figs. 3 and 4. It is preferable for the contrast marker(s) to be provided at an end portion or end portions of the embolus member. Particularly, it is preferable to provide the contrast markers respectively at both end portions of the embolus member. Specifically, as in the case of the embolus member 30 shown in Fig. 3 and Fig. 4 (development of the embolus member upon production), it is preferable that a plurality of contrast markers 39 are provided in the axial direction on one end side (specifically, on the distal end side), and a contrast marker 39 is provided on the other end side (specifically, on the proximal end side). This configuration makes it easy to check the positions of the end portions of the embolus member.

In the case shown in Fig. 3, the contrast marker is formed by a method in which a small opening formed in the embolus member is plugged up with a contrast marker, and the contrast marker is thereby fixed to the embolus member. Preferably, such a marker is attached, for example, by a method in which a disc form member of a contrast material slightly smaller than the small opening formed in the embolus member is placed in the small opening, and the disc form member is caulked by pressing it from both sides. Incidentally, the contrast marker is not limited to the above-mentioned one but may be any one. For example, there may be adopted a marker formed by coating an outside surface of the embolus member with a contrast material, a marker formed by winding a wire of a contrast material, a marker formed by attaching a ring form member of a contrast material, or the like. Incidentally, preferable examples of the material for forming the contrast markers include gold, platinum, tungsten, alloys thereof, and silver-palladium alloys. The contrast markers include those for radiography, those for sonography, those for NMR imaging, etc.

Besides, while the blood vessel embolus members in all possible constructions described above are of the so-called balloon expandable type, the blood vessel embolus members are not limited to this type but may be of the self-expandable type. In this case, the blood vessel embolus member has a structure in which one end portion is smaller in outside diameter than the other end portion, the one end portion and the other end portion are compressed in the direction of the center axis at the time of insertion into a blood vessel. At the time of indwelling in the blood vessel, they are expanded outwards to restore their shapes before compression. In such a blood vessel embolus member, the other end portion may have any shape that can be reduced in diameter at the time of insertion and can be enlarged in diameter (restored) upon release into a living body. Examples of the usable shape include a coil-like shape, a hollow cylindrical shape, a roll-like shape, an irregular tubular shape, a high-order coil-like shape, a leaf spring coil-like shape, and a cage- or mesh-like shape.

As the material for constituting the embolus member in this case, there may be used a synthetic resin or a metal. As the synthetic resin, one that has certain degrees of hardness and elasticity is used. Specific examples of the synthetic resin include polyolefins (e.g., polyethylene, polypropylene), polyesters (e.g., polyethylene terephthalate), and fluoro-resins (e.g., PTFE, ETFE). On the other hand, examples of the metal include stainless steels, tantalum, cobalt-based alloys, and super-elastic alloys.

Particularly, super-elastic alloys are used preferably. Here, the super-elastic alloys are those alloys generally called shape memory alloys and show super-elasticity at least at a living body temperature (around 37 degree C). Particularly, super-elastic alloys such as Ti-Ni alloys containing 49 to 53 at% Ni, Cu-Zn alloys containing 38.5 to 41.5 wt% Zn, Cu-Zn-X alloys (X = Be, Si, Sn, Al, Ga) containing 1 to 10 wt% X, and Ni-Al alloys containing 36 to 38 at% Al. Among these alloys, particularly preferred are the Ti-Ni alloys. Besides, the mechanical properties of the Ti-Ni alloys can be appropriately changed by substituting part of the Ti-Ni alloys by 0.01 to 10.0%X to obtain Ti-Ni-X alloys (X = Co, Fe, Mn, Cr, V, Al, Nb, W, B, or the like), by substituting part of the Ti-Ni alloys by 0.01 to 30.0 at% to obtain Ti-Ni-X alloys (X = Cu, Pb, Zr), or by selecting the cold working ratio and/or final heat treatment conditions. In addition, when the Ti-Ni-X alloy is used and the cold working rate and/or final heat treatment conditions are selected, the mechanical properties of the alloy can be appropriately changed.

The buckling strength (yield stress under a load) of the super-elastic alloys used here is in the range of 5 to 200 kg/mm² (22 degree C), preferably 8 to 150 kg/mm², and the restoring stress (yield stress under no load) of the super-elastic alloys is in the range of 3 to 180 kg/mm² (22 degree C), preferably 5 to 130 kg/mm². Herein, super-elasticity means that, even when the alloy is deformed (bent, extended, or compressed) into a region in which a usual metal is plastically deformed at the use temperature, the alloy will, upon release of the deformation, return substantially to the shape thereof before compression, without need for heating.

In the next place, a method of leaving a blood vessel embolus member to indwell in a blood vessel and a blood vessel occluding method, which does not form a part of the present invention, will be described below using the embolus member capturing device leaving implement 300 to which the embolus member capturing device 301 is mounted. Incidentally, in the following description, embolization of the gastroduodenal artery branched from the common hepatic artery will be taken as an example.

The method of leaving a blood vessel embolus member to indwell in a blood vessel includes: leaving an embolus member capturing device in a site of the blood vessel; and releasing an embolus member in the vicinity of and on the upstream side, with respect to the bloodstream, of the indwelling site for the embolus member capturing device to indwell in the blood vessel so as to capture the embolus member by the embolus member capturing device.

The blood vessel occluding method includes: leaving an embolus member capturing device in a site of a blood vessel; releasing an embolus member in the vicinity of and on the upstream side, with respective to the bloodstream, of the indwelling site for the embolus member capturing device to indwell in the blood vessel so as to capture the embolus member by the embolus member capturing device; and forming a thrombus on the surface of the embolus member or on the surfaces of the embolus member and the embolus member capturing device.

In the above-described method, the embolus member capturing device is left to indwell in the blood vessel in the state of having a small diameter portion on the downstream side with respect to the bloodstream in the blood vessel.

First, the process for leaving the embolus member capturing device to indwell in the occlusion site of a blood vessel is conducted.

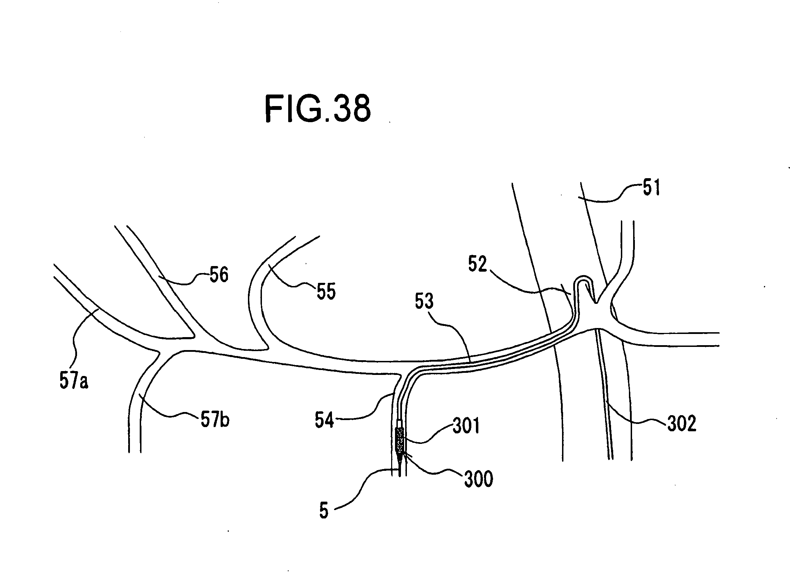

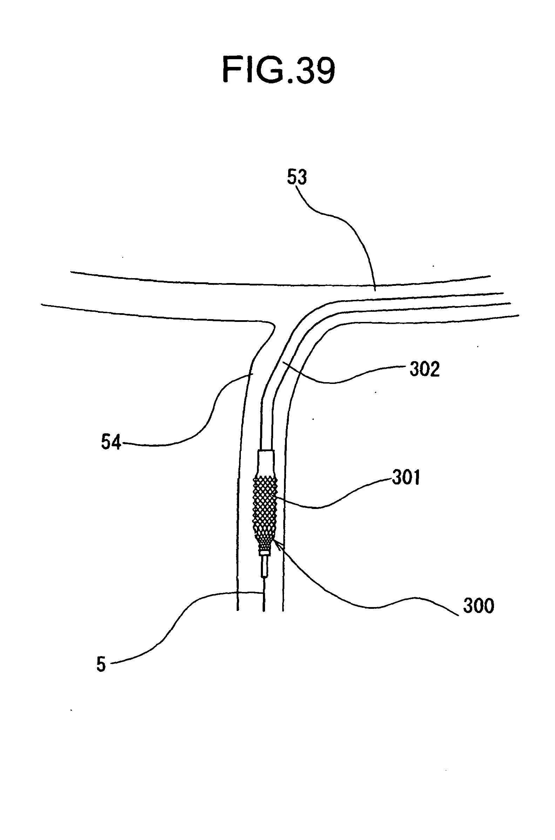

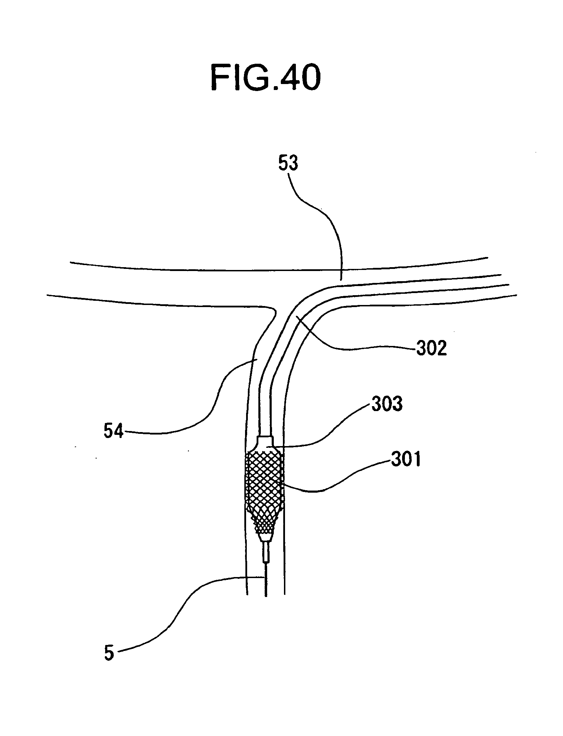

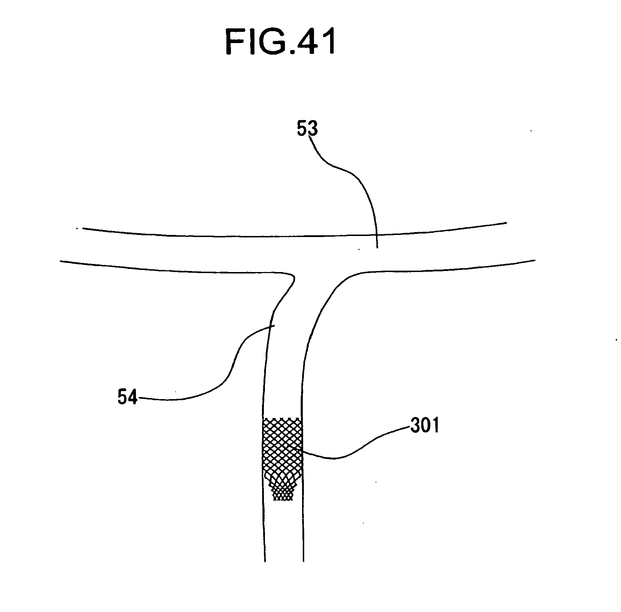

Specifically, as shown in Fig. 38, the embolus member capturing device leaving implement 300 with a guide wire 5 penetrating therethrough is inserted from the femoral artery (not shown). While causing the guide wire 5 to precede, the leaving implement 300 is made to go into the aorta abdominalis 51, is inserted through the celiac artery 52 (which branches from the aorta abdominalis 51) into the common hepatic artery 53, and is further pushed forwards to proceed into the gastroduodenal artery 54 (which branches from the common hepatic artery 53). Incidentally, on the peripheral side of the branching position of the gastroduodenal artery 54, the common hepatic artery 53 further branches through the proper hepatic artery to the left hepatic artery 55, the middle hepatic artery 56, and two right hepatic artery 57a, 57b. Then, the portion, where the embolus member capturing device 301 is mounted, of the embolus member capturing device leaving implement 300 is made to reach a desired site in the gastroduodenal artery 54. This condition is shown in Fig. 39. Then, a liquid for expansion is injected into the expansion body 303 of the leaving implement 300, to expand the expansion body 303. The expansion of the expansion body 303, as shown in Fig. 40, expands the embolus member capturing device 301 into the form having a small diameter portion on the peripheral side of the gastroduodenal artery 54. The device 301 is brought into close contact with the inside wall of the gastroduodenal artery 54, and is fixed to the artery 54. Then, as shown in Fig. 41, the catheter 302 of the embolus member capturing device leaving implement 300 is withdrawn together with the guide wire 5. As a result, the embolus member capturing device 301 expanded and deformed is left in a fixed state in the gastroduodenal artery 54.

Subsequently, an embolus member is released in the vicinity of and on the upstream side, with respect to the bloodstream, of the indwelling site for the embolus member capturing device 301 to indwell in the blood vessel.

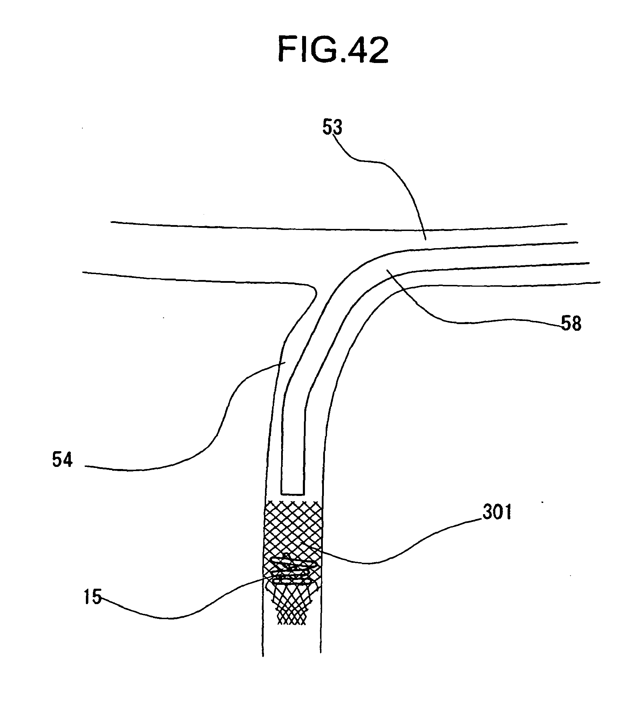

Specifically, a catheter 58 for supplying the embolus member is inserted into the gastroduodenal artery 54 while causing a guide wire (not shown) to precede. The insertion of the catheter 58 is conducted by pushing the catheter 58 forwards along the guide wire through the lumen of the catheter 58. By the forward movement of the catheter 58, a distal end portion of the catheter 58 reaches the vicinity or the inside of the embolus member capturing device 301. Under this condition, the guide wire is withdrawn. Subsequently, an embolus member 15 is inserted into the lumen via a proximal end portion of the catheter 58, the embolus member 15 is moved toward the distal side of the catheter 58 by a wire-like implement or a pusher, and the embolus member 15 is released via the distal end of the catheter 58. The embolus member 15 thus released is captured in the vicinity of the small diameter portion of the embolus member capturing device 301, as shown in Fig. 42.

Then, the process for forming a thrombus on the surface of the embolus member or on the surfaces of the embolus member and the embolus member capturing device is performed. This process needs no special operation; namely, this process proceeds by deposition or growth of thrombus on the surface of the embolus member left to indwell in the blood vessel or on the surfaces of the embolus member and the capturing device. Thereafter, the gastroduodenal artery 54 is occluded by the thrombus formed on the surface of the embolus member, so that the blood stops flowing into blood vessels on the peripheral side of the embolus member capturing device 301. Next, another catheter is inserted, and the distal end thereof is left to indwell in the common hepatic artery 53. When a therapeutic agent such as a carcinostatic is injected through the lumen of the catheter into the common hepatic artery 53, the therapeutic agent does not flow into the occluded gastroduodenal artery 54 but flows into the left hepatic artery and the like on the peripheral side.

Incidentally, the above description has been made by use of an possible variation of the type in which a blood vessel is occluded by both the embolus member and the embolus member capturing device. However, in the case of using an implement having a blood vessel occluding function such as the blood vessel embolus members 800, 850, 900, 950 described above, the above-mentioned operations for inserting the embolus member 15 and leaving the embolus member 15 to indwell in the blood vessel are unnecessary.

While preferred embodiments of the invention have been described using specific terms, such description is for illustrative purposes only, and it is to be understood that changes and variations may be made without departing from the scope of the following claims.

## Claims

1. An embolus member capturing device leaving implement (100) comprising a catheter (102) having an expandable and foldable expansion body (103) in the vicinity of a distal end portion, and an embolus member capturing device which is mounted to said catheter and is comprised of a tubular body (113) having one end portion and one other end portion, which is expandable when a radially relating force is entered from the inside of said tubular body, **characterized in that**
said expansion body (103) comprises a proximal side expandable portion, which is cylindrical and is expandable to a first outside diameter, a distal side expandable portion expandable to a second outside diameter smaller than said first outside diameter, and an intermediate expandable portion expandable to a shape reduced in diameter toward the distal side between said proximal side expandable portion and said distal side expandable portion, and said embolus member capturing device (101) is mounted to said expansion body in such a manner that said other end portion of said capturing device envelopes said proximal side expandable portion, said one end portion of said capturing device envelopes said distal side expandable portion, and the portion between said one end portion and said other end portion of said capturing device envelopes said intermediate expandable portion, and a proximal end of the other end portion of said embolus member capturing device is located at least on the proximal side relative to a central portion of said proximal side expandable portion.

2. The embolus member capturing device leaving implement as set forth in claim 1, further comprising a contrast marker (117, 118) provided on the one end side and/or the other end side.

3. The embolus member capturing device leaving implement as set forth in claims 1 or 2, wherein a part or the whole part of said embolus member capturing device is coated with a highly thrombus-generating material, or a fibrous member formed of a highly thrombus-generating material is attached to a part or the whole part of said embolus member capturing device (101).

4. The embolus member capturing device leaving implement as set forth in any of claims 1 to 3, wherein the outside diameter of said other end portion upon expansion of said embolus member capturing device can be regulated by an expansive force of said expansive body.

5. The embolus member capturing device leaving implement as set forth in any of claims 1 to 4, further comprising a protective sheath (205) for containing said catheter with said embolus member capturing device mounted.

## Patentansprüche

1. Gerät (100) zum Anbringen einer Embolusfangvorrichtung, das einen Katheter (102) mit einem dehnbaren und faltbaren Expansionskörper (103) im Bereich eines distalen Endabschnitts und eine Embolusfangvorrichtung umfasst, die an dem Katheter angebracht ist und aus einem rohrförmigen Körper (113) besteht, der einen Endabschnitt und einen anderen Endabschnitt besitzt, der dehnbar ist, wenn eine radial gerichtete Kraft von der Innenseite des rohrförmigen Körpers aus einwirkt, **dadurch gekennzeichnet, dass**
der Expansionskörper (103) einen proximalseitigen dehnbaren Abschnitt umfasst, der zylindrisch ist und auf einen ersten Außendurchmesser gedehnt werden kann, einen distalseitigen dehnbaren Abschnitt, der auf einen zweiten Außendurchmesser gedehnt werden kann, der kleiner ist als der erste Außendurchmesser, und einen mittleren dehnbaren Abschnitt zwischen dem proximalseitigen dehnbaren Abschnitt und dem distalseitigen dehnbaren Abschnitt, der zur distalen Seite hin zu einer Form von geringerem Durchmesser gedehnt werden kann, und dass die Embolusfangvorrichtung (101) so an dem Expansionskörper angebracht ist, dass der andere Endabschnitt der Fangvorrichtung den proximalseitigen dehnbaren Abschnitt umschließt, der eine Endabschnitt der Fangvorrichtung den distalseitigen dehnbaren Abschnitt umschließt und der Abschnitt zwischen dem einen Endabschnitt und dem anderen Endabschnitt der Fangvorrichtung den mittleren dehnbaren Abschnitt umschließt und sich ein proximales Ende des anderen Endabschnitts der Embolusfangvorrichtung mindestens auf der proximalen Seite in Bezug auf einen mittleren Abschnitt des proximalseitigen dehnbaren Abschnitts befindet.

2. Gerät zum Anbringen einer Embolusfangvorrichtung nach Anspruch 1, das ferner eine Kontrastmarkierung (117, 118) umfasst, die auf der einen Stirnseite und/oder der anderen Stirnseite vorgesehen ist.

3. Gerät zum Anbringen einer Embolusfangvorrichtung nach Anspruch 1 oder 2, wobei ein Teil oder der ganze Teil der Embolusfangvorrichtung mit einem stark thrombuserzeugenden Material beschichtet ist oder ein aus einem stark thrombuserzeugenden Material gebildetes Faserelement an einem Teil oder dem ganzen Teil der Embolusfangvorrichtung (101) befestigt ist.

4. Gerät zum Anbringen einer Embolusfangvorrichtung nach einem der Ansprüche 1 bis 3, wobei der Außendurchmesser des anderen Endabschnitts nach Aufdehnung der Embolusfangvorrichtung durch eine Dehnkraft des Expansionskörpers reguliert werden kann.

5. Gerät zum Anbringen einer Embolusfangvorrichtung nach einem der Ansprüche 1 bis 4, das ferner eine Schutzhülle (205) zur Aufnahme des Katheters mit der daran angebrachten Embolusfangvorrichtung umfasst.

## Revendications

1. Instrument (100) laissant un dispositif de capture d'élément d'embole, comprenant un cathéter (102) comportant un corps à expansion (103), expansible et pliable, situé au voisinage d'une partie d'extrémité distale, et un dispositif de capture d'élément d'embole qui est monté sur ledit cathéter et qui comprend un corps tubulaire (113) présentant une première partie terminale et une autre partie terminale, qui est expansible sous l'action d'une force dirigée radialement depuis l'intérieur dudit corps tubulaire, **caractérisé en ce que**
ledit corps à expansion (103) comprend une partie expansible de côté proximal, qui est cylindrique et qui peut s'expanser jusqu'à prendre un premier diamètre extérieur, une partie expansible de côté distal, qui peut s'expanser jusqu'à prendre un deuxième diamètre extérieur, plus petit que ledit premier diamètre extérieur, et une partie expansible intermédiaire qui peut s'expanser en une forme de diamètre réduit vers le côté distal et qui est située entre ladite partie expansible de côté proximal et ladite partie expansible de côté distal, et ledit dispositif (101) de capture d'élément d'embole est monté sur ledit corps expansible de telle manière que ladite autre partie terminale dudit dispositif de capture enveloppe ladite partie expansible de côté distal et la partie située entre ladite première partie terminale et ladite autre partie terminale dudit dispositif de capture enveloppe ladite partie expansible intermédiaire, et l'extrémité proximale de l'autre partie terminale dudit dispositif de capture d'élément d'embole est située au moins du côté proximal par rapport à la partie centrale de ladite partie expansible de côté proximal.

2. Instrument laissant un dispositif de capture d'élément d'embole, conforme à la revendication 1, qui comprend en outre un marqueur à contraste (117, 118) placé sur ladite premmière partie terminale et/ou ladite autre partie terminale.

3. Instrument laissant un dispositif de capture d'élément d'embole, conforme à la revendication 1 ou 2, dans lequel tout ou partie dudit dispositif de capture d'élément d'embole est revêtu d'un matériau qui favorise beaucoup la formation de thrombus, ou un élément fibreux fait d'un matériau qui favorise beaucoup la formation de thrombus est attaché à tout ou partie dudit dispositif de capture d'élément d'embole.

4. Instrument laissant un dispositif de capture d'élément d'embole, conforme à l'une des revendications 1 à 3, dans lequel le diamètre externe de ladite autre partie terminale après expansion dudit dispositif de capture d'élément d'embole peut être régulé par la force d'expansion agissant sur ledit corps à expansion.

5. Instrument laissant un dispositif de capture d'élément d'embole, conforme à l'une des revendications 1 à 4, qui comporte en outre une gaine protectrice (205) destinée à contenir ledit cathéter et ledit dispositif de capture d'élément d'embole monté dessus.
